# EUROPEAN PATENT APPLICATION

(11) **EP 4 691 456 A1**
(43) Date of publication of application: **11.02.2026**
(21) Application number: 24784716.3
(22) Date of filing: 19.03.2024
(51) Int. Cl.: A61K 8/898, A61K 8/894, A61Q 1/00, A61Q 1/06, A61Q 1/12, A61Q 5/06, A61Q 5/12, A61Q 17/04, A61Q 19/00

(54) **COSMETIC**

(30) Priority: 03.04.2023 JP 2023060187
(71) Applicant: SHIN-ETSU CHEMICAL CO., LTD., Tokyo 1000005 (JP)
(72) Inventor: IMAI Taro, Annaka-shi, Gunma 379-0224 (JP); KONISHI Masayuki, Tokyo 100-0005 (JP)
(74) Representative: Mewburn Ellis LLP
(86) International application number: PCT/JP2024/010633
(87) International publication number: WO 2024/209924

(57) **Abstract**

This cosmetic has excellent viscosity stability and contains (a) amino alcohol-modified silicone and (b) a polyglycerin-modified silicone surfactant.

## Description

### TECHNICAL FIELD

The present invention relates to a cosmetic containing an aminoalcohol-modified silicone. In the present invention, a composition for use in a cosmetic may be written as a cosmetic.

### BACKGROUND ART

Silicone surfactants are conventionally used widely as emulsifiers. In particular, polyglycerin-modified silicones show adhesiveness to the skin that is offered by the polyglycerin and have a light feel that is characteristic of silicones, thus finding wide use in emulsion cosmetics and the like.

Patent Document 1 describes a composition obtained by the addition reaction of a polyhydric alcohol-substituted hydrocarbon group having at least two hydroxyl groups and a silicone compound to an organohydrogenpolysiloxane, and proposes a method for synthesizing such a polyglycerin-modified silicone. However, polyglycerin-modified silicones have poor interfacial tension reducing ability, and this fact makes it difficult to reduce the emulsion particle size.

Patent Document 2 discloses a cosmetic that contains an aminoalcohol-modified organopolysiloxane as a silicone surfactant. This patent document also mentions the emulsion stability of the cosmetic including the aminoalcohol-modified organopolysiloxane. However, over-time changes in viscosity are not actually measured and the cosmetic is stable only as a result of the viscosity being increased by the addition of a large amount of silicone gelling agent or bentonite. Sufficient studies are not made as to whether the cosmetic including the aminoalcohol-modified organopolysiloxane will have emulsion stability when the viscosity is low.

### PRIOR ART DOCUMENTS

### PATENT DOCUMENTS

Patent Document 1: JP-A 2002-179798
Patent Document 2: JP-A 2015-113344

### SUMMARY OF INVENTION

### TECHNICAL PROBLEM

The present invention has been made in view of the circumstances discussed above. It is an object of the present invention to provide a cosmetic having excellent viscosity stability and, when in the form of emulsion, having a small emulsion particle size.

### SOLUTION TO PROBLEM

As a result of extensive studies directed to achieving the above object, the present inventors have found that the problems described above can be solved by using a combination of (a) an aminoalcohol-modified silicone and (b) a polyglycerin-modified silicone surfactant. The present invention has been completed based on the finding.

Thus, the present invention provides the following cosmetics.
1. A cosmetic including:
   (a) an aminoalcohol-modified silicone and
   (b) a polyglycerin-modified silicone surfactant.
2. The cosmetic according to 1, wherein the aminoalcohol-modified silicone (a) is an aminoalcohol-modified silicone represented by formula (1) below: 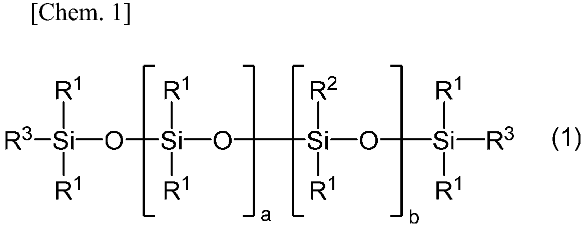
   [R¹ independently at each occurrence is a monovalent hydrocarbon group selected from C1-C20 alkyl groups, C6-C15 aryl groups, and C7-C15 aralkyl groups; R² independently at each occurrence is a group represented by formula (2) below: 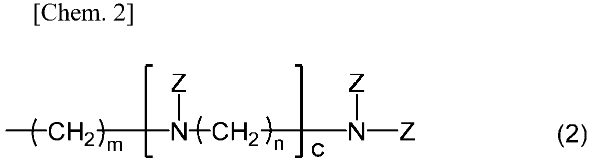
   (Z independently at each occurrence is a group selected from hydrogen atom, -CH₂CH(OH)CH₃, and -CH(CH₃)CH₂OH; 50 mol% or more of a number of moles of all Zs in the molecule are groups selected from -CH₂CH(OH)CH₃ and -CH(CH₃)CH₂OH; m is an integer of 1 to 10; and n is an integer of 1 to 5); R³ is R¹ or R²; a is an integer of 1 to 100; b is an integer of 0 to 10; c is an integer of 0 to 4; and when b = 0, at least one of R³s is R²].
3. The cosmetic according to 2, wherein in formula (1), R² is selected from aminoalcohol groups represented by formulas (3) and (4) below: 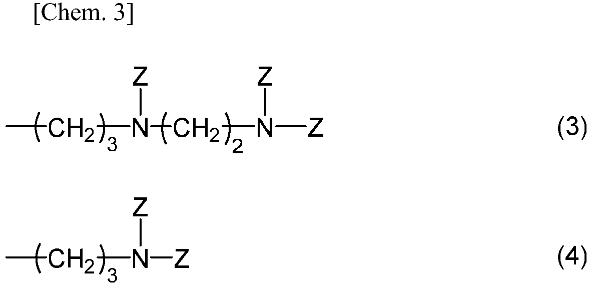 (wherein Z is the same as defined above).
4. The cosmetic according to 2, wherein in formula (1), b is an integer of 1 to 10, and a/b is 10 to 30.
5. The cosmetic according to 2, wherein in formula (1), 99 to 100 mol% of the number of moles of all Zs in the molecule are -CH₂CH(OH)CH₃ or -CH(CH₃)CH₂OH.
6. The cosmetic according to any one of 1 to 5, wherein the ingredient (b) is a partially crosslinked polyglycerin-modified silicone surfactant.

### ADVANTAGEOUS EFFECTS OF INVENTION

The cosmetic of the present invention exhibits excellent viscosity stability over time even when having a low viscosity, and, in the case where the cosmetic is an emulsion, has a small emulsion particle size. The cosmetic can exhibit excellent viscosity stability over time even when the cosmetic contains an ingredient that is detrimental to emulsion stability, such as ethanol.

### DESCRIPTION OF EMBODIMENTS

The present invention will be described in described in detail below. The present invention will be described in detail below. In the present invention, the name of an ingredient is written as the name cited in *Kesh* 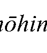 *hin Hy* 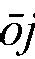 *ji Meish* 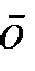 [Japanese Cosmetic Labeling Names] or in the International Nomenclature of Cosmetic Ingredients (INCI). When the names from *Kesh* 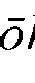 *hin Hy* 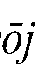 *ji Meish* 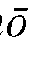 and the INCI are the same, the name from *Kesh* 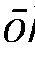 *hin Hy* 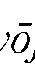 *ji Meish* 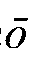 or the INCI name is sometimes omitted.

### [Ingredients (a)]

The ingredient (a) in the present invention is an aminoalcohol-modified silicone. The aminoalcohol-modified silicones may be used singly, or two or more may be used in combination. Examples of the aminoalcohol-modified silicones include aminoalcohol-modified silicones represented by formula (1) below: 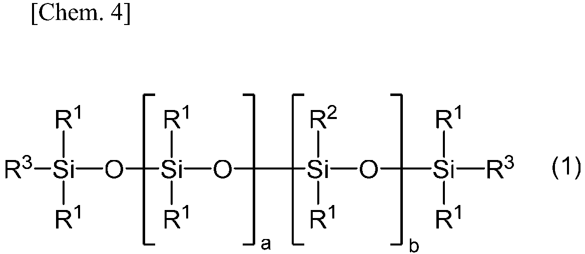
[R¹ independently at each occurrence is a monovalent hydrocarbon group selected from C1-C20 alkyl groups, C6-C15 aryl groups, and C7-C15 aralkyl groups; R² independently at each occurrence is a group represented by formula (2) below: 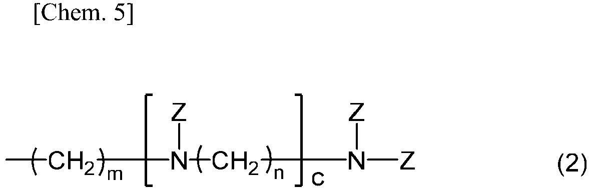
(Z independently at each occurrence is a group selected from hydrogen atom, -CH₂CH(OH)CH₃, and -CH(CH₃)CH₂OH; 50 mol% or more of the number of moles of all Zs in the molecule are groups selected from -CH₂CH(OH)CH₃ and -CH(CH₃)CH₂OH; m is an integer of 1 to 10; and n is an integer of 1 to 5); R³ is R¹ or R²; a is an integer of 1 to 100; b is an integer of 0 to 10; c is an integer of 0 to 4; and when b = 0, at least one of R³s is R²]. In the present invention, the order in which the siloxane units are bonded is not limited to that illustrated in the formula.

R¹ independently at each occurrence is a monovalent hydrocarbon group selected from C1-C20 alkyl groups, C6-C15 aryl groups, and C7-C15 aralkyl groups. Specific examples include alkyl groups, such as methyl group, ethyl group, propyl group, butyl group, and pentyl group; cycloalkyl groups, such as cyclopentyl group; and aryl groups, such as phenyl group and tolyl group. R¹ is preferably a methyl group or a phenyl group, and more preferably a methyl group.

R² independently at each occurrence is a group represented by formula (2) below: 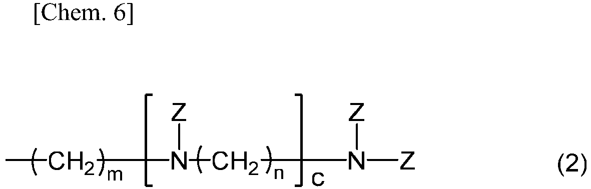 (Z independently at each occurrence is a group selected from hydrogen atom, -CH₂CH(OH)CH₃, and -CH(CH₃)CH₂OH; 50 mol% or more of the number of moles of all Zs in the molecule are groups selected from -CH₂CH(OH)CH₃ and -CH(CH₃)CH₂OH; m is an integer of 1 to 10; and n is an integer of 1 to 5).

In formula (2), 50 mol% or more of the number of moles of all Zs in the molecule are groups selected from -CH₂CH(OH)CH₃ and -CH(CH₃)CH₂OH. This proportion is preferably 90 mol% or more, and more preferably 99 to 100 mol%. The letter m is an integer of 1 to 10, and preferably 1 to 5. The letter n is an integer of 1 to 5, and preferably 1 to 3. By virtue of the above proportion being 50 mol% or more, the compound is capable of allowing small particles to be emulsified and the over-time discoloration stemming from amino groups can be reduced.

R³ is R¹ or R² described above. The letter a is 1 to 100, preferably 10 to 80, and more preferably 20 to 60. The letter b is 0 to 10, preferably 1 to 10, and more preferably 1 to 6. The letter c is an integer of 0 to 4, and more preferably 0 to 1. When b = 0, at least one of R³s is R². It is preferable that the letter b be an integer of 1 to 10, and a/b be 10 to 30, more preferably 10 to 20. In particular, formula (1) is preferably such that R² is a group selected from formulas (3) and (4) below: 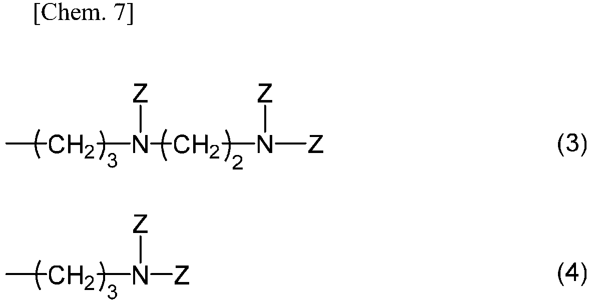 (wherein Z is the same as defined above). Furthermore, it is preferable that 99 mol% or more of the number of moles of all Zs in the molecule be groups selected from -CH₂CH(OH)CH₃ and -CH(CH₃)CH₂OH.

The kinematic viscosity of the ingredient (a) at 25°C is preferably 100 to 5,000 mm²/s, and more preferably 200 to 4,000 mm²/s. The kinematic viscosity is a value measured at 25°C using a Cannon-Fenske viscometer as described in JIS Z8803.

The amount in which the ingredient (a) is added is preferably 0.1 to 15 mass%, more preferably 0.2 to 10 mass%, still more preferably 0.5 to 8 mass%, and particularly preferably 0.5 to 5 mass% of the whole cosmetic. When the amount added is 0.1 mass% or more, the emulsion stability is enhanced. Adding more than 15 mass% may result in a strong sticky feeling.

The production method is not particularly limited, and the ingredient (a) may be obtained by reacting an organopolysiloxane having an amino group with ethylene oxide, propylene oxide, or glycidol.

### (b) Polyglycerin-modified silicone surfactants

The ingredient (b) in the present invention is a polyglycerin-modified silicone surfactant. Specific examples include partially crosslinked polyglycerin-modified silicones, linear or branched polyglycerin-modified organopolysiloxanes, and linear or branched polyglycerin/alkyl-co-modified organopolysiloxanes. In particular, partially crosslinked polyglycerin-modified silicones that are linear dimethylpolysiloxane crosslinked with polyglycerin may be suitably used in terms of emulsion stability.

Examples of the partially crosslinked polyglycerin-modified silicones include (Dimethicone/Polyglycerin-3) Crosspolymer (INCI), (Lauryl Dimethicone/Polyglycerin-3) Crosspolymer (INCI), and (Polyglycerin-3/Lauryl Polydimethylsiloxyethyl Dimethicone) Crosspolymer (INCI). The partially crosslinked polyglycerin-modified silicone may be a composition consisting of the partially crosslinked polyglycerin-modified silicone and an oil that is liquid at room temperature. Examples of the liquid oils include Cyclopentasiloxane (INCI), Dimethicone (INCI), Mineral Oil (INCI), Isododecane (INCI), Triethylhexanoin (INCI), and Squalane (INCI).

Exemplary commercial products in which a partially crosslinked polyglycerin-modified silicone is swollen by containing a liquid oil include KSG-710, KSG-810, KSG-820, KSG-830, KSG-840, KSG-820Z, and KSG-850Z manufactured by Shin-Etsu Chemical Co., Ltd.

Specific examples of the linear or branched polyglycerin-modified organopolysiloxanes and the linear or branched polyglycerin/alkyl-co-modified organopolysiloxanes include Polyglyceryl-3 Disiloxane Dimethicone (INCI), Polyglyceryl-3 Polydimethylsiloxyethyl Dimethicone (INCI), Lauryl Polyglyceryl-3 Polydimethylsiloxyethyl Dimethicone (INCI), and Bis-butyl Dimethicone Polyglyceryl-3 (INCI). Exemplary commercial products include KF-6100, KF-6104, KF-6105, KF-6106, and KF-6115 manufactured by Shin-Etsu Chemical Co., Ltd.

The ingredients (b) may be used singly, or two or more that differ from one another may be used in combination. In any case, the amount thereof is preferably 0.1 to 4 mass%, more preferably 0.2 to 3 mass%, still more preferably 0.6 to 2 mass%, and particularly preferably 0.8 to 1.5 mass% based on the whole cosmetic. When the amount is 0.1 mass% or more, a stable emulsion can be obtained more reliably. When the amount is 4 mass% or less, a cosmetic with a light feel can be obtained.

In the present invention, the mass ratio a/b is preferably 0.5 to 30, more preferably 0.7 to 4, and still more preferably 0.7 to 3.5.

### [Cosmetics]

The present invention is applicable to a variety of cosmetics, particularly preferably to cosmetics externally applied to the skin, such as skin care cosmetics, makeup cosmetics, antiperspirant cosmetics, and UV-protecting cosmetics, to cosmetics externally applied to the hair, such as hair care cosmetics, and to nail cosmetics. Exemplary skin care cosmetics include skin lotions, milky lotions, creams, cleansing agents, packs, oil liquids, massage creams, cosmetic liquids, cosmetic oils, detergents, deodorants, hand creams, lip creams, and anti-wrinkle agents.

Exemplary makeup cosmetics include makeup bases, foundations, concealers, cosmetic powders, cheek colors, eye colors, eye shadows, mascaras, eye liners, eyebrows, and lip cosmetics. Exemplary antiperspirant cosmetics include antiperspirants of roll-on, cream, solution, and stick types. Exemplary UV-protecting cosmetics include sunscreen oils, sunscreen milky lotions, and sunscreen creams. Exemplary hair care cosmetics include shampoos, conditioners, treatments, and setting agents.

The type of the cosmetic of the present invention may be any of, for example, powder, oily liquid, water-in-oil emulsion, oil-in-water emulsion, non-aqueous emulsion, and multi-phase emulsion, such as W/O/W or O/W/O. The form of the cosmetic of the present invention may be selected from various forms including liquid, milky lotion, cream, solid, paste, gel, powder, pressed, multilayer, mousse, spray, stick, and pencil.

Among those described above, emulsions are preferable, and water-in-oil emulsions are more preferable. Water-in-oil emulsions may be viscous or solid.

When the cosmetic is a viscous cosmetic, the viscosity at 25°C is preferably 5,000 to 200,000 mPa·s. According to the present invention, it is possible to achieve stabilization of not only medium-viscosity and high-viscosity emulsions but also of low-viscosity emulsions. In the present invention, low-viscosity emulsions are those having a viscosity of less than 20,000 mPa·s, or a viscosity of 15,000 mPa·s or less. The lower limit is not particularly limited but may be 100 mPa·s. The viscosity is a value measured at 25°C using a Brookfield viscometer in accordance with the method described in JIS K 7117-1: 1999. For example, the Brookfield viscometer may be TVB-10 manufactured by Toki Sangyo Co., Ltd.

In the case of emulsions, the emulsion particle size is preferably less than 4.0 µm, and more preferably less than 2.0 µm. In the present invention, the emulsion particle size is the average of the sizes of 20 emulsion particles determined by observation of the cosmetic with an optical microscope.

The cosmetic of the present invention may contain various ingredients that are usually added to cosmetics without impairing the advantageous effects of the present invention. For example, such additional ingredients are (1) oils, (2) aqueous ingredients, (3) surfactants other than the ingredients (b), (4) powders, (5) compositions consisting of a crosslinked organopolysiloxane and an oil that is liquid at room temperature, (6) film-forming agents, (7) UV-absorbing/scattering agents, and (8) other additives. These may be each used singly, or two or more may be used in appropriate combination. The amounts of these ingredients may be selected appropriately in accordance with the type of cosmetic. The amount of the oil (1) or the aqueous ingredient (2) may be the balance.

### (1) Oils

The oils may be volatile or nonvolatile and may be solid, semisolid, or liquid at room temperature (25°C). Examples include silicone oils, silicone waxes, natural animal and plant oils and fats, semi-synthetic oils and fats, hydrocarbon oils, higher alcohols, fatty acids, ester oils, fluorochemical oils, and UV absorbers.

### • Silicone oils

Examples of the silicone oils include Dimethicone (INCI); Trisiloxane (INCI); alkyl-modified silicones, such as Methyl Trimethicone (INCI), Ethyl Trisiloxane (INCI), Ethyl Methicone (INCI), and Hexyl Dimethicone (INCI); long-chain alkyl-modified silicones, such as Caprylyl Methicone (INCI); low- to high-viscosity, linear or branched organopolysiloxanes, such as Phenyl Trimethicone (INCI), Diphenyl Dimethicone (INCI), Diphenylsiloxyphenyl Trimethicone (INCI), Tetraphenyl Dimethyldisiloxane (INCI), and Methylhydrogenpolysiloxane; cyclic organopolysiloxanes, such as Cyclotetrasiloxane (INCI), Cyclopentasiloxane (INCI), and Cyclohexasiloxane (INCI); amino-modified organopolysiloxanes, such as Amodimethicone (INCI) and Aminopropyl Dimethicone (INCI); pyrrolidone-modified organopolysiloxanes, such as PCA Dimethicone (INCI); pyrrolidone carboxylic acid-modified organopolysiloxanes; silicone rubbers, such as gum-like dimethylpolysiloxanes having a high degree of polymerization, gum-like amino-modified organopolysiloxanes, and gum-like dimethylsiloxane-methylphenylsiloxane copolymers; as well as low-viscosity organopolysiloxane solutions of silicone gums and rubbers; amino acid-modified silicones; fluorine-modified silicones; silicone resins; and silicone resin solutions. Examples of commercially available silicone oils include KF-96L-1cs, KF-96L-1.5cs, KF-96L-2cs, KF-96A-6cs, KF-4422, KF-4418, TMF-1.5, KF-54, KF-54HV, KF-56A, and KF-995 manufactured by Shin-Etsu Chemical Co., Ltd.

### • Solid oil ingredients

When a solid cosmetic is desired in the present invention, it is preferable to add an oil ingredient that is solid at 25°C. The oil ingredient that is solid at 25°C is preferably one having a melting point of 40°C or above, more preferably 60 to 110°C. Examples include waxes, hydrocarbons, esters, higher alcohols, and higher fatty acids. The solid oil ingredients are not particularly limited and may be any raw materials usually added to cosmetics. Specific examples include plant waxes, such as carnauba wax (INCI: Copernicia Cerifera (Carnauba) Wax), sugar cane wax, candelilla wax (INCI: Euphorbia Cerifera (Candelilla) Wax), purified candelilla wax, rice wax, Japan wax, jojoba wax, kapok wax, rice bran wax, myrica cerifera fruit wax, shea butter, cacao butter, Japan wax (INCI: Rhus Succedanea Fruit Wax), montan wax (INCI: Montan Wax), and hydrogenated castor oil isostearate; animal waxes, such as beeswax, beef tallow, beef bone fat, lard (INCI: Lard), horse fat (INCI: Horse Fat), mutton tallow, lanolin (INCI: Lanolin), Ericerus pela wax, shellac wax, and sperm wax; semi-synthetic waxes, such as lanolin esters, lanolin fatty acid esters, and beeswax acid esters; hydrogenated oils, such as hydrogenated castor oil and hydrogenated coconut oil; hydrocarbon waxes, such as solid paraffin, polyethylene, ceresin, ozokerite, and microcrystalline wax; wax esters, such as synthetic beeswax; amino acid stearyl alcohols, such as dioctyldodecyl lauroylglutamate, dioctyldodecyl lauroylglutamate, and dioctyldodecyl lauroylglutamate; fatty acids, such as stearic acid and behenic acid; silicone waxes, such as acrylate silicone resins in the form of acrylate silicone graft or block copolymers (such as acrylate silicone graft copolymers: KP-561P and KP-562P manufactured by Shin-Etsu Chemical Co., Ltd.); and derivatives thereof. Any one, or two or more selected from the foregoing are preferable.

### • Natural animal and plant oils and semi-synthetic oils

Examples of the natural animal and plant oils and the semi-synthetic oils include natural plant oils, such as avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), perilla oil (labeling name), olive oil (labeling name, INCI: Olea Europaea (Olive) Fruit Oil), Torreya californica oil (labeling name, INCI: Torreya Californica (California Nutmeg) Oil), citronella grass oil (labeling name, INCI: Cymbopogon Nardus (Citronella) Oil), Torreya Nucifera seed oil (labeling name, INCI: Torreya Nucifera Seed Oil), apricot kernel oil (labeling name, INCI: Kyounin Yu), wheat germ oil (labeling name, INCI: Triticum Vulgare (Wheat) Germ Oil), sesame oil (labeling name, INCI: Sesamum Indicum (Sesame) Seed Oil), rice germ oil (labeling name, INCI: Oryza Sativa (Rice) Germ Oil), rice bran oil (labeling name, INCI: Oryza Sativa (Rice) Bran Oil), sasanqua oil (labeling name, INCI: Camellia Kissi Seed Oil), safflower oil (labeling name, INCI: Carthamus Tinctorius (Safflower) Seed Oil), soybean oil (labeling name, INCI: Glycine Soja (Soybean) Oil), tea seed oil (labeling name, INCI: Camellia Sinensis Seed Oil), camellia oil (labeling name, INCI: Camellia Japonica Seed Oil), evening primrose oil (labeling name, INCI: Oenothera Biennis (Evening Primrose) Oil), rapeseed oil (labeling name), germ oils, for example, corn germ oil (labeling name, INCI: Zea Mays (Corn) Germ Oil), persic oil (labeling name), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), sunflower oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia seed oil (labeling name, INCI: Macadamia Ternifolia Seed Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), and peanut oil (labeling name, INCI: Arachis Hypogaea (Peanut) Oil); natural animal oils, such as shark liver oil (labeling name, INCI: Shark Liver Oil), cod liver oil (labeling name, INCI: Cod Liver Oil), fish liver oil (labeling name, INCI: Fish Liver Oil), turtle oil (labeling name, INCI: Turtle Oil), mink oil (labeling name, INCI: Mink Oil), and egg yolk oil (labeling name, INCI: Egg Oil); and semi-synthetic oils and fats, such as hydrogenated coconut oil (labeling name, INCI: Hydrogenated Coconut Oil) and liquid lanolin (labeling name, INCI: Lanolin Oil).

### • Hydrocarbon oils

Examples of the hydrocarbon oils include linear or branched hydrocarbon oils. The hydrocarbon oils may be volatile or nonvolatile. Specific examples include alkanes, such as Olefin Oligomers (INCI), isoparaffins, for example, (C13, 14) Isoparaffins (INCI), Isododecane (INCI), Undecane (INCI), Dodecane (INCI), Isohexadecane (INCI), hydrogenated polyisobutene (labeling name, INCI: Hydrogenated Polyisobutene), Squalane (INCI), Mineral Oil (INCI), Coconut Alkanes (INCI), and (C13-15) Alkanes (INCI).

### • Higher alcohols

Examples of the higher alcohols include linear saturated alcohols having 6 or more carbon atoms, such as Lauryl Alcohol (INCI), Hexyldecanol (INCI), Oleyl Alcohol (INCI), Isostearyl Alcohol (INCI), Octyldodecanol (INCI), Decyltetradecanol (INCI), Myristyl Alcohol (INCI), Cetyl Alcohol (INCI), Stearyl Alcohol (INCI), and Behenyl Alcohol (INCI), as well as Batyl Alcohol (INCI). Examples further include sterols, such as Cholesterol (INCI), sitosterol (labeling name, INCI: Beta-Sitosterol)), Phytosterols (INCI), and Lanosterol (INCI).

### • Ester oils

Examples of the ester oils include diisobutyl adipate (labeling name, INCI: Diisobutyl Adipate), dihexyldecyl adipate (labeling name), diheptylundecyl adipate (labeling name, INCI: Diheptylundecyl Adipate), alkylglycol monoisostearates, such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), octyldodecyl esters, such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol dioctanoate (labeling name, INCI: Neopentyl Glycol Diethylhexanoate), neopentyl glycol dicaprate (labeling name, INCI: Neopentyl Glycol Dicaprate), diisostearyl malate (labeling name, INCI: Diisostearyl Malate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), palmitates, such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl (labeling name, INCI: Cholesteryl Hydroxystearate), myristates, such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), isopropyl lauroyl sarcosinate (labeling name, INCI: Isopropyl Lauroyl Sarcosinate), and coco-alkyl caprylate/caprate (labeling name, INCI: Coco-Caprylate/Caprate).

Among the ester oils, exemplary glyceride oils include Triethylhexanoin (INCI), glyceryl tri(caprylate/caprate) (labeling name, INCI: Caprylic/Capric Triglyceride), Cocoglyceryl (INCI), triglyceryl caprylate/caprate/succinate (labeling name, INCI: Caprylic/Capric/Succinic Triglyceride), and caprylic/capric glycerides (labeling name, INCI: Caprylic/Capric Glycerides).

### • Fluorochemical oils

Examples of the fluorochemical oils include Perfluorodecalin (INCI), Perfluorononyl Dimethicone (INCI), and Perfluoromethylcyclopentane (INCI).

### • UV absorbers

Examples of the UV absorbers include oxybenzone-1 (labeling name, INCI: Benzophenone-1), oxybenzone-2 (labeling name, INCI: Benzophenone-2), oxybenzone-3 (labeling name, INCI: Benzophenone-3), oxybenzone-4 (labeling name, INCI: Benzophenone-4), oxybenzone-5 (labeling name, INCI: Benzophenone-5), oxybenzone-6 (labeling name, INCI: Benzophenone-6), oxybenzone-9 (labeling name, INCI: Benzophenone-9), Homosalate (INCI), Octocrylene (INCI), t-butyl methoxydibenzoylmethane (labeling name, INCI: Butyl Methoxydibenzoylmethane), ethylhexyl salicylate (labeling name, INCI: Ethylhexyl Salicylate), diethylaminohydroxybenzoyl hexyl benzoate (labeling name, INCI: Diethylamino Hydroxybenzoyl Hexyl Benzoate), Polysilicone-15 (INCI), dimethoxybenzylidene dioxoimidazolidine octyl propionate (labeling name, INCI: Ethylhexyl Dimethoxybenzylidene Dioxoimidazolidine Propionate), terephthalylidene dicamphor sulfonic acid (labeling name, INCI: Terephthalylidene Dicamphor Sulfonic Acid), Ethylhexyl Triazone (INCI), methylbis(trimethylsiloxy)silyl isopentyl trimethoxycinnamate (labeling name, INCI: Isopentyl Trimethoxycinnamate Trisiloxane), Drometrizole Trisiloxane (INCI), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), Bis-Ethylhexyloxyphenol Methoxyphenyltriazine (INCI), phenylbenzimidazole sulfonic acid (labeling name, INCI: Phenylbenzimidazole Sulfonic Acid), Methylene Bis-Benzotriazolyl Tetramethylbutylphenol (INCI), glyceryl ethylhexanoate dimethoxycinnamate (labeling name, INCI: Glyceryl Ethylhexanoate Dimethoxycinnamate), Glyceryl PABA (INCI), methyl diisopropylcinnamate (labeling name, INCI: Diisopropyl Methyl Cinnamate), Cinoxate (INCI), and ethylhexyl dimethoxybenzylidene dioxoimidazolidine propionate (labeling name, INCI: Ethylhexyl Dimethoxybenzylidene Dioxoimidazolidine Propionate). Furthermore, a UVA absorber (such as, for example, Diethylamino Hydroxybenzoyl Hexyl Benzoate) and a UVB absorber (such as, for example, Ethylhexyl Methoxycinnamate) may be used in combination, or absorbers belonging to the same type may be combined appropriately.

### (2) Aqueous ingredients

The aqueous ingredients are not particularly limited as long as they are aqueous ingredients that are usually added to cosmetics. Specific examples include water, lower alcohols preferably having 2 to 5 carbon atoms, such as ethanol (labeling name, INCI: Alcohol) and isopropanol (labeling name, INCI: Isopropyl Alcohol), and sugar alcohols, such as Sorbitol (INCI), Maltose (INCI), and Xylitol (INCI). Examples further include polyhydric alcohols, such as BG (labeling name, INCI: Butylene Glycol), PG (labeling name, INCI: Propylene Glycol), DPG (labeling name, INCI: Dipropylene Glycol), Pentylene Glycol (INCI), 1,10-Decanediol (INCI), Octanediol (INCI), 1,2-Hexanediol (INCI), Erythritol (INCI), Glycerin (INCI), Diglycerin (INCI), and polyethylene glycol; and moisturizers, such as Glucose (INCI), Glyceryl Glucoside (INCI), Betaine (INCI), Na chondroitin sulfate (labeling name, INCI: Sodium Chondroitin Sulfate), PCA-Na (labeling name, INCI: Sodium PCA), Methyl Gluceth-10 (INCI), Methyl Gluceth-20 (INCI), hyaluronic acid, egg yolk lecithin, soybean lecithin, phosphatidylcholine, phosphatidylethanolamine, phosphatidylserine, phosphatidylglycerol, phosphatidylinositol, and sphingophospholipid.

Ethanol as an aqueous ingredient generally deteriorates the emulsion stability of a water-in-oil emulsion cosmetic. However, the addition of the ingredients (a) and (b) of the present invention can stabilize such a cosmetic. Ethanol is useful to adjust the feeling on use and to solubilize various hardly soluble ingredients. Thus, the availability of ethanol expands the variety of cosmetics that can be prepared. The amount in which ethanol is added is preferably 0.1 to 20 mass%, and more preferably 0.5 to 15 mass% of the whole cosmetic, and is still more preferably 1 to 10 mass% in terms of the feeling on use.

### (3) Surfactants other than the ingredients (b)

The surfactants may be nonionic, anionic, cationic, or ampholytic. The surfactants are not particularly limited and any surfactants usually used in cosmetics may be used. Among such surfactants, one, or two or more selected from non-crosslinked silicone surfactants and crosslinked silicone surfactants are preferable because stable cosmetics can be obtained. For any types of surfactants, the amount is preferably 0.1 to 20 mass% of the whole of the cosmetic. An amount of 0.1 mass% or more ensures that the dispersing and emulsifying functions are fully achieved whereas an amount of 20 mass% or less advantageously eliminates the risk that the cosmetic gives a sticky feeling on use. The HLB of the surfactants is not limited but is preferably 2 to 14.5 in order to maintain the water resistance of the cosmetic.

The non-crosslinked silicone surfactants other than the ingredients (b) are linear or branched silicones in which some methyl groups on the backbone are substituted with hydrophilic groups, such as polyethylene glycol. Specifically, preferred non-crosslinked silicone surfactants are linear or branched polyoxyethylene-modified organopolysiloxanes, linear or branched polyoxyethylene polyoxypropylene-modified organopolysiloxanes, linear or branched polyoxyethylene/alkyl-co-modified organopolysiloxanes, linear or branched polyoxyethylene polyoxypropylene/alkyl-co-modified organopolysiloxanes, and linear or branched pyrrolidone-modified organopolysiloxanes. Examples include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), and Cetyl PEG/PPG-10/1 Dimethicone (INCI).

Examples of commercially available products include KF-6011, KF-6011P, KF-6012, KF-6015, KF-6017, KF-6043, KF-6028, KF-6038, and KF-6048 manufactured by Shin-Etsu Chemical Co., Ltd.

Examples of the crosslinked silicone surfactants other than the ingredients (b) include crosslinked polyether-modified silicones, such as (Dimethicone/(PEG-10/15)) Crosspolymer (INCI), (PEG-15/Lauryl Dimethicone) Crosspolymer (INCI), (PEG-10/Lauryl Dimethicone) Crosspolymer (INCI), and (PEG-15/Lauryl Polydimethylsiloxyethyl Dimethicone) Crosspolymer (INCI).
When the crosslinked silicone surfactant is used as a composition consisting of the crosslinked silicone surfactant and an oil that is liquid at room temperature, it is preferable that the crosslinked silicone surfactant be swollen by containing its own weight or more of the liquid oil. The liquid oil may be a liquid silicone oil, a hydrocarbon oil, an ester oil, a natural animal or plant oil, a semi-synthetic oil, or a fluorochemical oil, with examples including Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI).

Commercially available crosslinked silicone surfactants swollen by containing a liquid oil include, for example, KSG-210, KSG-240, KSG-270, KSG-310, KSG-320, KSG-330, KSG-340, KSG-320Z, and KSG-350Z manufactured by Shin-Etsu Chemical Co., Ltd.

### (4) Powders

Examples of the powders include coloring pigments, inorganic powders, metal powders, organic powders, and inorganic-organic composite powders. These powders are described in detail below.

### • Coloring pigments

The coloring pigments are not particularly limited and may be any pigments commonly used in cosmetics for the purpose of coloring. Examples include red iron oxide (labeling name, INCI: Iron Oxides), yellow iron oxide (labeling name, INCI: Iron Oxides), white titanium dioxide (labeling name, INCI: Titanium Dioxide), black iron oxide (labeling name, INCI: Iron Oxides), ultramarine (labeling name, INCI: Ultramarines), Prussian blue (labeling name, INCI: Ferric Ferrocyanide, Ferric Ammonium Ferrocyanide), manganese violet (labeling name, INCI: Manganese Violet), cobalt titanate (labeling name, INCI: Cobalt Titanium Dioxide), chromium hydroxide (labeling name, INCI: Chromium Hydroxide Green), chromium oxide (labeling name, INCI: Chromium Oxide Greens), Al/cobalt oxide (labeling name, INCI: Cobalt Aluminum Oxide), fired titanium/titanium dioxide (labeling name, INCI: Titanium/Titanium Dioxide), Li/cobalt titanate (labeling name, INCI: Lithium Cobalt Titanate), sintered iron oxide/titanium dioxide (labeling name), composites doped with a different metal, such as iron oxide-doped titanium dioxide (labeling name, INCI: Iron Oxides, Titanium Dioxide), titanium nitride (labeling name, INCI: Titanium Nitride), ferrous hydroxide (labeling name, INCI: Iron Hydroxide), inorganic brown pigments, such as γ-iron oxide, inorganic yellow pigments, such as loess, and colored pigments, such as lake form of tar dyes and lake form of natural dyes. Any of the foregoing may be used. The pigments may take any shape, with examples including spherical, generally spherical, bar, spindle, petal, strip, and irregular shapes. Their geometry is not particularly limited as long as the pigments can impart a color to the cosmetics.

### • Inorganic powders

Examples of the inorganic powders include microparticles of zirconium oxide (labeling name, INCI: Zirconium Dioxide), zinc oxide (labeling name, INCI: Zinc Oxide), cerium oxide (labeling name, INCI: Cerium Oxide), Mg oxide (labeling name, INCI: Magnesium Oxide), Ba sulfate (labeling name, INCI: Barium Sulfate), calcium sulfate (labeling name, INCI: Calcium Sulfate), Mg sulfate (labeling name, INCI: Magnesium Sulfate), Ca carbonate (labeling name, INCI: Calcium Carbonate), Mg carbonate (labeling name, INCI: Magnesium Carbonate), Talc (INCI), Mica (INCI), Kaolin (INCI), synthetic fluorphlogopite (labeling name, INCI: Synthetic Fluorphlogopite), synthetic ferrous phlogopite (labeling name), black mica (labeling name, INCI: Biotite), K silicate (labeling name, INCI: Potassium Silicate), Silica (INCI), Al silicate (labeling name, INCI: Aluminum Silicate), Mg silicate (labeling name, INCI: Magnesium Silicate), Al/Mg silicate (labeling name, INCI: Magnesium Aluminum Silicate), Ca silicate (labeling name, INCI: Calcium Silicate), Al/Ca/Na silicate (labeling name, INCI: Aluminum Calcium Sodium Silicate), Li/Mg/Na silicate (labeling name, INCI: Lithium Magnesium Sodium Silicate), Na/Mg silicate (labeling name, INCI: Sodium Magnesium Silicate), Ca/Al borosilicate (labeling name, INCI: Calcium Aluminum Borosilicate), Ca/Na borosilicate (labeling name, INCI: Calcium Sodium Borosilicate), Hydroxyapatite (INCI), Bentonite (INCI), Montmorillonite (INCI), Hectorite (INCI), Zeolite (INCI), Alumina (INCI), Al hydroxide (labeling name, INCI: Aluminum Hydroxide), boron nitride (labeling name, INCI: Boron Nitride), and glass (labeling name, INCI: Glass).
Furthermore, examples of the inorganic coloring pearly pigments include pearly agents, such as Mica (INCI) coated with titanium dioxide (labeling name, INCI: Titanium Dioxide) and synthetic fluorphlogopite (labeling name, INCI: Synthetic Fluorphlogopite) coated with titanium dioxide (labeling name, INCI: Titanium Dioxide); and pearly pigments, such as bismuth oxychloride (labeling name, INCI: Bismuth Oxychloride), bismuth oxychloride (labeling name, INCI: Bismuth Oxychloride) coated with titanium dioxide (labeling name, INCI: Titanium Dioxide), Talc (INCI) coated with titanium dioxide (labeling name, INCI: Titanium Dioxide), fish scale flakes (labeling name), and coloring mica coated with titanium dioxide (labeling name, INCI: Titanium Dioxide). These may be untreated or may have undergone well-known surface treatment generally used for cosmetic products.

### • Metal powders

Examples of the metal powders include metal microparticles of Al (labeling name, INCI: Aluminum, Aluminum Powder), copper (labeling name, INCI: Copper Powder), silver (labeling name, INCI: Silver Powder), and gold (labeling name, INCI: Gold).

### • Organic powders

Examples of the organic powders include powders of silicones, polyamides, polyacrylic acids/acrylates, polyesters, Polyethylene (INCI), Polypropylene (INCI), Polystyrene (INCI), styrene/acrylic acid copolymers, divinylbenzene/styrene copolymers, polyurethanes, vinyl resins, urea resins, melamine resins, benzoguanamine, polymethyl benzoguanamine, tetrafluoroethylene, polymethyl methacrylate, Cellulose (INCI), Silk (INCI), nylon (labeling name), phenolic resins, epoxy resins, and polycarbonates.

In particular, examples of the silicones include silicone resin particles; Polymethylsilsesquioxane (INCI), silicone rubber powders, silicone resin-coated silicone rubber powders; vinyl dimethicone/methicone silsesquioxane crosspolymer (labeling name, INCI: Vinyl Dimethicone/Methicone Silsesquioxane Crosspolymer), diphenyl dimethicone/vinyldiphenyl dimethicone/silsesquioxane crosspolymer (labeling name, INCI: Diphenyl Dimethicone/Vinyl Diphenyl Dimethicone/Silsesquioxane Crosspolymer), Polysilicone-1 Crosspolymer (INCI), and Polysilicone-22 (INCI).

Examples of commercially available silicone powders include KMP-590, KMP-591, KMP-592, KMP-597, KMP-598, KSP-100, KSP-101, KSP-102, KSP-105, KSP-300, KSP-411, KSP-441, KM-9729, and KM-440 manufactured by Shin-Etsu Chemical Co., Ltd.

Examples further include powders of metal soaps, specifically, zinc stearate (labeling name, INCI: Zinc Stearate), Al stearate (labeling name, INCI: Aluminum Stearate), Ca stearate (labeling name, INCI: Calcium Stearate), Mg stearate (labeling name, INCI: Magnesium Stearate), zinc myristate (labeling name, INCI: Zinc Myristate), Mg myristate (labeling name, INCI: Magnesium Myristate), zinc/Na cetylphosphate (labeling name, INCI: Sodium Zinc Cetyl Phosphate), and K cetylphosphate (labeling name, INCI: Potassium Cetyl Phosphate).

Examples further include organic colorants, specifically, tar dyes, such as Red #3, Red #104(1) (labeling name, INCI: Red 28, Red 28 Lake), Red #106, Red #201 (labeling name, INCI: Red 6), Red #202 (labeling name, INCI: Red 7), Red #204, Red #205, Red #220 (labeling name, INCI: Red 34), Red #226 (labeling name, INCI: Red 30), Red #227 (labeling name, INCI: Red 33, Red 33 Lake), Red #228 (labeling name, INCI: Red 36), Red #230(1) (labeling name, INCI: Red 22, Red 22 Lake), Red #230(2) (labeling name), Red #401 (labeling name), Red #505 (labeling name), Yellow #4 (labeling name, INCI: Yellow 5), Yellow #5 (labeling name, INCI: Yellow 6, Yellow 6 Lake), Yellow #202(1) (labeling name, INCI: Yellow 8), Yellow #203 (labeling name, INCI: Yellow 10, Yellow 10 Lake), Yellow #204 (labeling name, INCI: Yellow 11), Yellow #401, Blue #1 (labeling name, INCI: Blue 1, Blue 1 Lake), Blue #2, Blue #201, Blue #205 (labeling name, INCI: Blue 4), Blue #404 (labeling name), Green #3 (labeling name, INCI: Green 3, Green 3 Lake), Green #201 (labeling name, INCI: Green 5), Green #202 (labeling name, INCI: Green 6), Green #204 (labeling name, INCI: Green 8), Green #205 (labeling name), Orange #201 (labeling name, INCI: Orange 5), Orange #203 (labeling name, INCI: Pigment Orange 5), Orange #204 (labeling name), Orange #205 (labeling name, INCI: Orange 4, Orange 4 Lake), Orange #206 (labeling name, INCI: Orange 10), and Orange #207 (labeling name, INCI: Orange 11); and natural dyes, such as Cochineal (INCI), laccaic acid (labeling name, INCI: Laccaic Acid), safflower red (labeling name, INCI: Carthamus Tinctorius (Safflower) Flower Extract), red root extract (labeling name, INCI: Lithospermum Officinale Root Extract), gardenia yellow (labeling name), and gardenia blue (labeling name, INCI: Hydrolyzed Gardenia Florida Extract).

### • Inorganic/organic composite powders

Examples of the inorganic/organic composite powders include composite powders obtained by coating the surface of an inorganic powder with an organic powder by any well-known method.

The powders described hereinabove may be surface-treated. From the point of view of the water resistance of the cosmetic, the surface treatment agent is preferably one that can impart hydrophobicity. The hydrophobicity-imparting agent is not particularly limited, and examples thereof include silicone treatment agents, waxes, paraffins, organic fluorine compounds, such as perfluoroalkyl phosphates, surfactants, amino acids, such as N-acylglutamic acid, and metal soaps, such as aluminum stearate and magnesium myristate. Silicone treatment agents are more preferable. Examples thereof include silanes or silylating agents, such as Triethoxycaprylylsilane (INCI), Dimethicone (INCI), Methicone (INCI), Hydrogen Dimethicone (INCI), Triethoxysilylethyl Polydimethylsiloxyethyl Dimethicone (INCI), Triethoxysilylethyl Polydimethylsiloxyethylhexyl Dimethicone (INCI), and acrylates/tridecyl acrylate/triethoxysilylpropyl methacrylate/dimethicone methacrylate copolymer (labeling name, INCI: Acrylates/Tridecyl Acrylate/Triethoxysilylpropyl Methacrylate/Dimethicone Methacrylate Copolymer). Specific examples of the silicone treatment agents include AES-3083, KF-99P, KF-9901, KF-9908, KF-9909, KP-574, and KP-541 manufactured by Shin-Etsu Chemical Co., Ltd. The surface hydrophobizing agents may be used singly, or two or more may be used in combination. Specific examples of the surface-treated coloring pigments include KTP-09 series, in particular, KTP-09W, 09R, 09Y, and 09B manufactured by Shin-Etsu Chemical Co., Ltd.

### (5) Compositions consisting of a crosslinked organopolysiloxane and an oil that is liquid at room temperature

In the composition consisting of a crosslinked organopolysiloxane and an oil that is liquid at room temperature, the crosslinked organopolysiloxane is preferably swollen by containing its own weight or more of the liquid oil. The liquid oil may be a liquid silicone oil, a hydrocarbon oil, an ester oil, a natural animal or plant oil, a semi-synthetic oil, or a fluorochemical oil. Examples include Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI). Unlike the ingredients (b) and the crosslinked silicone surfactants belonging to the ingredients (3), the ingredients (5) are compounds that do not have a polyether or polyglycerin structure in the molecular structure. Specific examples include Dimethicone/Vinyl Dimethicone Crosspolymer (INCI), dimethicone/phenyl vinyl dimethicone crosspolymer (labeling name, INCI: Dimethicone/Phenyl Vinyl Dimethicone Crosspolymer), Vinyl Dimethicone/Lauryl Dimethicone Crosspolymer (INCI), and Lauryl Polydimethylsiloxyethyl Dimethicone/Bis-Vinyl Dimethicone Crosspolymer (INCI).

Examples of the commercial compositions consisting of a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-042Z, KSG-045Z, and KSG-048Z manufactured by Shin-Etsu Chemical Co., Ltd.

### (6) Film-forming agents

The film-forming agent is added mainly for the purpose of further extending the durability of the effects of the cosmetic. The film-forming agents are not particularly limited. Silicone compositions are preferable from the point of view of imparting water repellency. Specifically, for example, use may be made of trimethylsiloxysilicic acid, acrylic-silicone film formers, silicone-modified norbornene, silicone-modified pullulan, and silicone-modified polyvinyl alcohol. Examples of the film-forming agents in the form of silicone compositions include trimethylsiloxysilicic acid (labeling name, INCI: Trimethylsiloxysilicate), Acrylates/Dimethicone Copolymer (INCI), Norbornene/Tris(Trimethylsiloxy)Silylnorbornene Copolymer (INCI), and pullulan tri(trimethylsiloxy)silylpropyl carbamide (labeling name, INCI: Trimethylsiloxysilylcarbamoyl Pullulan). The film-forming agent may be previously dissolved into an oil that is liquid at room temperature before being added to the cosmetic. The liquid oil may be a liquid silicone oil, a hydrocarbon oil, an ester oil, a natural animal or plant oil, a semi-synthetic oil, or a fluorochemical oil.

Specific examples of commercially available silicone film-forming agents include KF-7312J, KP-545, KP-549, KP-543, NBN-30-ID, TSPL-30-ID, and TSPL-30-D5 manufactured by Shin-Etsu Chemical Co., Ltd.

### (7) UV-absorbing/scattering agents

Examples of the UV-absorbing/scattering agents include particles that absorb and scatter ultraviolet rays, such as titanium dioxide microparticles, iron-containing titanium dioxide microparticles, zinc oxide microparticles, cerium oxide microparticles, and composites thereof. These UV-absorbing/scattering particles may be used as a dispersion in an oil. The oil may be a liquid silicone oil, a hydrocarbon oil, an ester oil, a natural animal or plant oil, a semi-synthetic oil, or a fluorochemical oil. Specific examples of the oil dispersions of the UV-absorbing/scattering particles include SPD series (product name), in particular, SPD-T5, T5L, Z5, Z5L, T6, Z6, T7, and Z7L manufactured by Shin-Etsu Chemical Co., Ltd.

### (8) Other additives

Other additives include oil-soluble gelling agents, preservatives, antibacterial agents, antiperspirants, fragrances, salts, antioxidants, acidity regulators, chelating agents, refreshing agents, anti-inflammatory agents, skin modifying ingredients (such as brightening or whitening agents, cell activators, anti-skin-roughening agents, blood flow enhancing agents, skin astringents, and antiseborrheic agents), vitamins, amino acids, nucleic acids, hormones, and clathrate compounds.

### • Oil-soluble gelling agents

Exemplary oil-soluble gelling agents include metal soaps, such as aluminum stearate, magnesium stearate, and zinc myristate; amino acid derivatives, such as lauroyl glutamic acid (labeling name, INCI: Lauroyl Glutamic Acid) and α,γ-di-n-butylamine; dextrin fatty acid esters, such as dextrin palmitate (labeling name, INCI: Dextrin Palmitate), dextrin isostearate (labeling name, INCI: Dextrin Isostearate), dextrin myristate (labeling name, INCI: Dextrin Myristate), inulin stearate (labeling name, INCI: Stearoyl Inulin), and dextrin palmitate/ethylhexanoate (labeling name, INCI: Dextrin Palmitate/Ethylhexanoate); sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; organo-modified clay minerals of hectorite, such as Disteardimonium Hectorite (INCI) and Stearalkonium Hectorite (INCI); and Stearalkonium Bentonite (INCI).

### • Preservatives and antibacterial agents

Examples of the preservatives and the antibacterial agents include alkyl p-hydroxybenzoates, benzoic acid, sodium benzoate, sorbic acid, potassium sorbate, phenoxyethanol, imidazolidinyl urea, salicylic acid, isopropyl methyl phenol, phenol, p-chloro-m-cresol, hexachlorophene, benzalkonium chloride, chlorohexidine chloride, trichlorocarbanilide, iodopropynyl butylcarbamate, polylysine, photosensitizers, silver, and plant extracts.

### • Antiperspirants

Examples of the antiperspirants include aluminum halohydrates, such as aluminum chlorohydrate, aluminum halides, such as aluminum chloride, aluminum allantoinate, tannic acid, persimmon tannin, aluminum potassium sulfate, zinc oxide, zinc p-phenolsulfonate, calcined alum, aluminum zirconium tetrachlorohydrate, and aluminum zirconium trichlorohydrex glycine. In particular, preferred ingredients that exert a higher effect include aluminum halohydrates, aluminum halides, and complexes or mixtures thereof with zirconium oxyhalides and zirconium hydroxyhalides (for example, aluminum zirconium tetrachlorohydrate and aluminum zirconium trichlorohydrex glycine).

### • Fragrances

The fragrances include natural fragrances and synthetic fragrances. Examples of the natural fragrances include plant fragrances separated from flowers, leaves, stems, and pericarps; and animal fragrances, such as musk and civet. Examples of the synthetic fragrances include hydrocarbons, such as monoterpene; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

### • Salts

Examples of the salts include inorganic salts, organic acid salts, amine salts, and amino acid salts. Exemplary inorganic salts include sodium salts, potassium salts, magnesium salts, calcium salts, aluminum salts, zirconium salts, and zinc salts of inorganic acids, such as hydrochloric acid, sulfuric acid, carbonic acid, and nitric acid. Exemplary organic acid salts include salts of organic acids, such as acetic acid, dehydroacetic acid, citric acid, malic acid, succinic acid, ascorbic acid, and stearic acid. Exemplary amine salts and amino acid salts include salts of amines, such as triethanol amine, and salts of amino acids, such as glutamic acid. Also useful are salts of hyaluronic acid, chondroitin sulfate, aluminum zirconium glycine complex, and acid-alkali neutral salts used in cosmetic formulations.

### • Antioxidants

Examples of the antioxidants include, but are not particularly limited to, carotenoid, ascorbic acid and salts thereof, ascorbyl stearate, tocopherol, tocopherol acetate, tocopherol, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin.

### • Acidity regulators

Examples of the acidity regulators include lactic acid, citric acid, glycolic acid, succinic acid, tartaric acid, dl-malic acid, potassium carbonate, sodium hydrogencarbonate, and ammonium hydrogencarbonate.

### • Chelating agents

Examples of the chelating agents include alanine, sodium EDTA, sodium polyphosphate, sodium metaphosphate, and phosphoric acid.

### • Refreshing agents

Examples of the refreshing agents include L-menthol, camphor, and menthyl lactate.

### • Anti-inflammatory agents

Examples of the anti-inflammatory agents include allantoin, glycyrrhizinic acid and salts thereof, glycyrrhetinic acid, stearyl glycyrrhetinate, tranexamic acid, and azulene.

### • Skin modifying ingredients

Examples of the skin modifying ingredients include brightening or whitening agents, such as placenta extract, arbutin, glutathione, and Saxifraga Sarmentosa Extract; cell activators, such as royal jelly, photosensitizers, cholesterol derivatives, and bovine blood extracts; anti-skin-roughening agents; blood flow enhancing agents, such as vanillylamide nonylate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents, such as zinc oxide and tannic acid; and antiseborrheic agents, such as sulfur and thianthol.

### • Vitamins

Examples of the vitamins include vitamin A family, such as vitamin A oil, retinol, retinol acetate, and retinol palmitate; vitamin B family, specifically, vitamin B2 family, such as riboflavin, riboflavin butyrate, and flavin adenine nucleotide, vitamin B6 family, such as pyridoxine hydrochloride, pyridoxine dioctanoate, and pyridoxine tripalmitate, vitamin B12 and derivatives thereof, and vitamin B15 and derivatives thereof; vitamin C family, such as L-ascorbic acid, L-ascorbic acid dipalmitic acid ester, L-ascorbic acid-2-sodium sulfate, and dipotassium L-ascorbic acid phosphoric acid diester; vitamin D family, such as ergocalciferol and cholecalciferol; vitamin E family, such as α-tocopherol, β-tocopherol, γ-tocopherol, dl-α-tocopherol acetate, dl-α-tocopherol nicotinate, and dl-α-tocopherol succinate; nicotinic acids, such as nicotinic acid, benzyl nicotinate, and nicotinic amide; vitamin H; vitamin P; pantothenic acids, such as calcium pantothenate, D-pantothenyl alcohol, pantothenyl ethyl ether, and acetylpantothenyl ethyl ether; and biotin.

### • Amino acids

Examples of the amino acids include glycine, valine, leucine, isoleucine, serine, threonine, phenylalanine, arginine, lysine, aspartic acid, glutamic acid, cystine, cysteine, methionine, and tryptophan.

### • Nucleic acids

Examples of the nucleic acids include deoxyribonucleic acid.

### • Hormones

Examples of the hormones include estradiol and ethenyl estradiol.

### • Clathrate compounds

Examples of the clathrate compounds include cyclodextrin.

### EXAMPLES

The present invention will be described in detail below by presenting Production Examples, and Examples and Comparative Examples of cosmetics. However, it should be construed that the present invention is not limited to the following Examples. Unless otherwise specified, the compositional "%" described below is "mass%" and means the mass percentage of the ingredient relative to the mass of the whole in each Example taken as 100 mass%. The amounts are the amounts of the products described. In the following Production Examples 1 to 11, the kinematic viscosity is a value measured at 25°C using a Cannon-Fenske viscometer as described in JIS Z8803.

### [Production Example 1]

An autoclave was charged with 100 parts by mass of an amino-modified silicone represented by average chemical formula (5) below, 20 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 1,000 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or N(CH(CH₃)-CH₂OH).

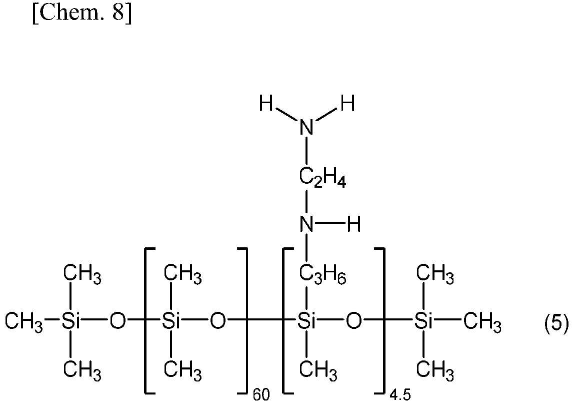

### [Production Example 2]

An autoclave was charged with 100 parts by mass of an amino-modified silicone represented by average chemical formula (6) below, 11 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 510 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or N(CH(CH₃)-CH₂OH).

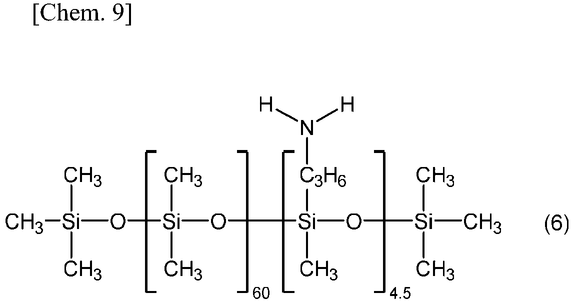

### [Production Example 3]

An autoclave was charged with 100 parts by mass of an amino-modified silicone represented by average chemical formula (7) below, 20 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 900 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or N(CH(CH₃)-CH₂OH).

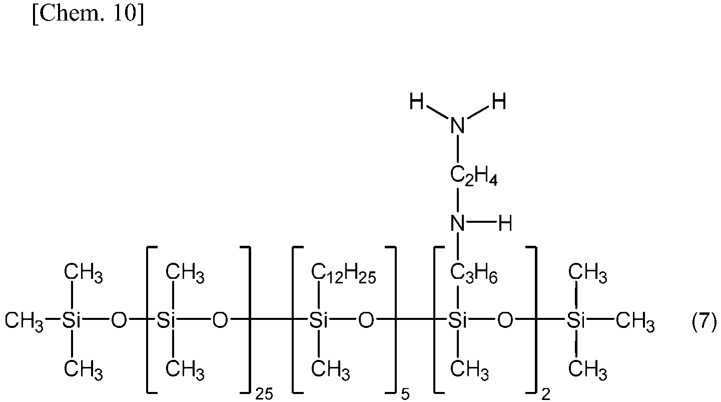

### [Production Example 4]

An autoclave was charged with 100 parts by mass of an amino-modified silicone represented by average chemical formula (8) below, 8 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 3,200 mm²/s was obtained. ¹H-NMR (CDCl₃, 400 MHz, Bruker) measurement confirmed that 60 mol% of the -NH₂ groups had been converted to -N(CH₂-CH(OH)-CH₃) or N(CH(CH₃)-CH₂OH).

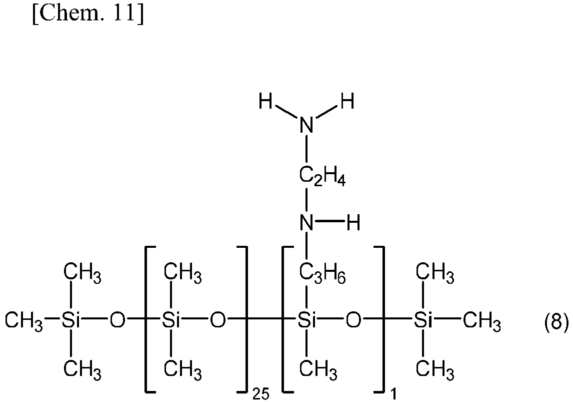

### [Production Example 5]

An autoclave was charged with 100 parts by mass of an amino-modified silicone represented by average chemical formula (9) below, 10 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 1,300 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or N(CH(CH₃)-CH₂OH).

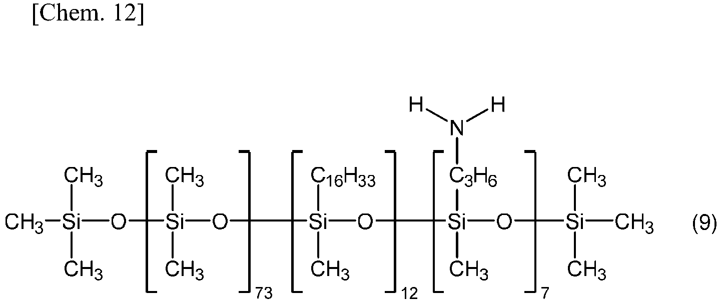

### [Production Example 6]

An autoclave was charged with 100 parts by mass of an amino-modified silicone represented by average chemical formula (10) below, 11 parts by mass of propylene oxide, and 4 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 1,800 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or N(CH(CH₃)-CH₂OH).

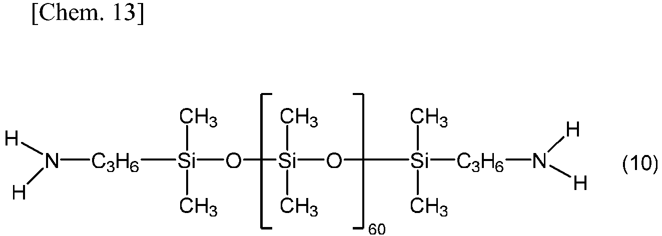

### [Production Example 7]

An autoclave was charged with 100 parts by mass of an amino-modified silicone represented by average chemical formula (11) below, 60 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 1,100 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or N(CH(CH₃)-CH₂OH).

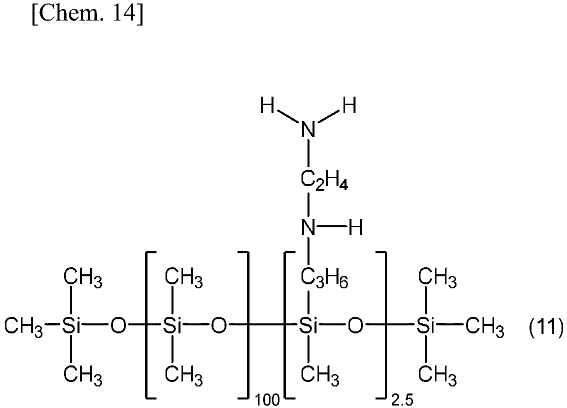

### [Production Example 8]

An autoclave was charged with 100 parts by mass of an amino-modified silicone represented by average chemical formula (12) below, 14 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 760 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or N(CH(CH₃)-CH₂OH).

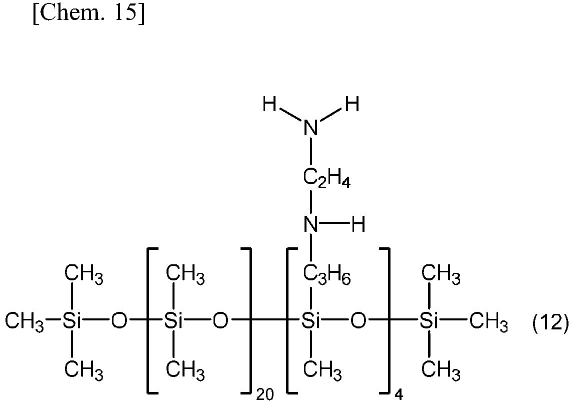

### [Production Example 9]

An autoclave was charged with 100 parts by mass of an amino-modified silicone represented by average chemical formula (13) below, 26 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 480 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or N(CH(CH₃)-CH₂OH).

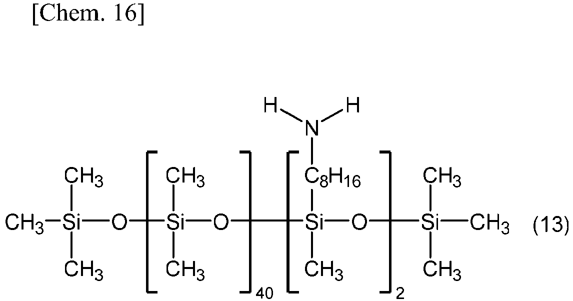

### [Production Example 10]

An autoclave was charged with 100 parts by mass of an amino-modified silicone represented by average chemical formula (14) below, 20 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 1,500 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or N(CH(CH₃)-CH₂OH).

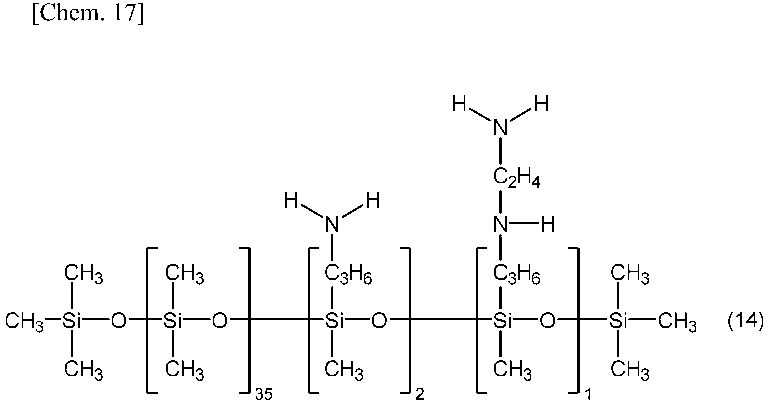

### [Production Example 11]

An autoclave was charged with 100 parts by mass of an amino-modified silicone represented by average chemical formula (15) below, 20 parts by mass of propylene oxide, and 6 parts by mass of hexylene glycol. The mixture was heated at 130°C for 6 hours. Unreacted materials were stripped from the resultant reaction mixture under reduced pressure at 130°C. Thus, a pale yellow, transparent liquid with a kinematic viscosity of 1,500 mm²/s was obtained. ¹H-NMR (CDCl₃, 400 MHz, Bruker) measurement confirmed that at least 99 mol% of the -NH₂ groups had been converted to -N(CH₂-CH(OH)-CH₃) or -N(CH(CH₃)-CH₂-OH).

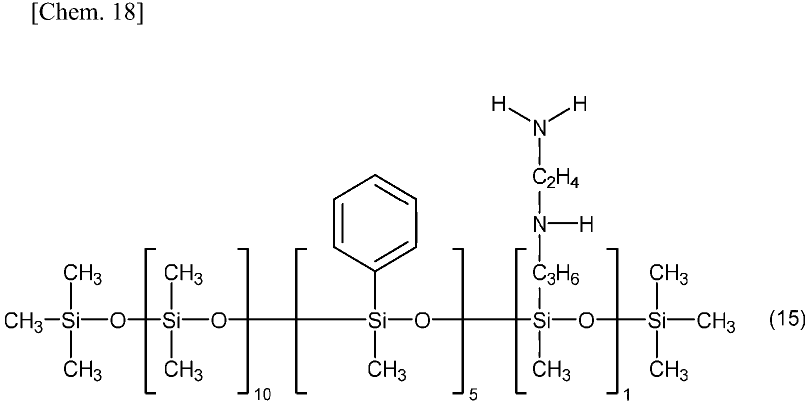

The advantageous effects of the present invention will be demonstrated below by way of Examples and Comparative Examples related to cosmetics.

### [Examples 1 to 3, and Comparative Examples 1 to 5]

Water-in-oil (W/O) cosmetics were prepared according to the formulations described in Table 1. The water-in-oil (W/O) cosmetics obtained were evaluated as follows.

### (Production method)

A: Ingredients (1) were mixed.
B: Ingredients (2) were mixed.
C: The mixture obtained in B was added to the mixture obtained in A and emulsified to give a water-in-oil (W/O) cosmetic. The viscosities of the cosmetics of Examples and Comparative Examples immediately after preparation were low in the range of 5,000 to 15,000 mPa·s. The viscosity for this evaluation was measured at 25°C using a Brookfield viscometer (TVB-10, manufactured by Toki Sangyo Co., Ltd.) with spindle No. 4, at 6 rpm for a measurement time of 60 seconds.

### [Emulsion particle size]

The cosmetic obtained was observed under an optical microscope, and the sizes of 20 emulsion particles were measured and averaged. The rating was "A" when the average particle size was less than 2.0 µm, "B" when the average particle size was 2.0 µm or more and less than 4.0 µm, "C" when the average particle size was 4.0 µm or more and less than 6.0 µm, and "D" when the average particle size was 6.0 µm or more. Those cosmetics that had "B" or higher rating were accepted.

### [Stability]

The cosmetic was added into a 30 mL vial and stored in a thermostatic chamber at 50°C for one week. The viscosity was then measured.

The viscosity after storage (the post-storage viscosity) was compared to the viscosity immediately after preparation (the initial viscosity) and the viscosity change (%) was calculated from (post-storage viscosity/initial viscosity - 1) × 100. The stability was rated as "A" when the viscosity change was 0% or more and less than ±15%, "B" when the viscosity change was ±15% or more and less than ±20%, "C" when the viscosity change was ±20% or more and less than ±30%, and "D" when the cosmetic had separated or the viscosity change was ±30% or more. Those cosmetics that had "B" or higher rating were accepted.

**[Table 1]**

| Composition (%) | | Example | | | Comparative Example | | | | |
|---|---|---|---|---|---|---|---|---|---|
| | | 1 | 2 | 3 | 1 | 2 | 3 | 4 | 5 |
| (1) | (a) Production Example 1 | 2 | - | - | 2 | - | - | - | - |
| | (a) Production Example 2 | - | 2 | 2 | - | 2 | - | - | - |
| | (b) KSG-710 (Note 1) | 3 | 3 | - | - | - | 3 | 3 | 5 |
| | KF-6017 (Note 2) | - | - | - | - | - | 2 | - | - |
| | (b) KF-6104 (Note 3) | - | - | 1 | - | - | - | 2 | - |
| | KF-96A-6cs (Note 4) | 25 | 25 | 27 | 28 | 28 | 25 | 25 | 25 |
| (2) | Sodium chloride | 0.5 | 0.5 | 0.5 | 0.5 | 0.5 | 0.5 | 0.5 | 0.5 |
| | Butylene Glycol | 5 | 5 | 5 | 5 | 5 | 5 | 5 | 5 |
| | Water | 64.5 | 64.5 | 64.5 | 64.5 | 64.5 | 64.5 | 64.5 | 64.5 |
| Total | | 100 | 100 | 100 | 100 | 100 | 100 | 100 | 100 |
| (a)/(b) | | 0.7 | 0.7 | 2.0 | - | - | - | - | - |
| Emulsion particle size | | A | B | B | A | B | B | D | D |
| Initial viscosity (mPa·s) | | 9,700 | 10,200 | 9,800 | 11,000 | 12,500 | 6,200 | 6,400 | 12,000 |
| Post-storage viscosity (mPa·s) | | 10,800 | 10,900 | 8,000 | 7,200 | 7,500 | 2,200 | 2,400 | 7,800 |
| Viscosity change (%) | | 11 | 7 | -18 | -35 | -40 | -65 | -63 | -35 |
| Stability | | A | A | B | D | D | D | D | D |

| | | | | | | | | | |
|---|---|---|---|---|---|---|---|---|---|
| Production Example 1: Aminoalcohol-modified silicone obtained in Production Example 1 Production Example 2: Aminoalcohol-modified silicone obtained in Production Example 2 (Note 1) Mixture of 70-80% dimethicone (6 cs) + 20-30% (dimethicone/polyglycerin-3) crosspolymer, Shin-Etsu Chemical Co., Ltd. (Note 2) PEG-10 dimethicone (comparative product), Shin-Etsu Chemical Co., Ltd. (Note 3) Polyglyceryl-3 polydimethylsiloxyethyl dimethicone, Shin-Etsu Chemical Co., Ltd. (Note 4) Dimethicone (6 cs), Shin-Etsu Chemical Co., Ltd. | | | | | | | | | |

As is clear from Table 1, the cosmetics of Examples 1 to 3 had a small emulsion particle size and had good viscosity stability in spite of the fact that they were low-viscosity emulsions that were heretofore difficult to stabilize.

### [Examples 4 and 5, and Comparative Examples 6 and 7]

Water-in-oil (W/O) cosmetics were prepared according to the formulations described in Table 2. The water-in-oil (W/O) cosmetics obtained were evaluated as follows.

### (Production method)

A: Ingredients (1) were mixed.
B: Ingredients (2) were mixed.
C: The mixture obtained in B was added to the mixture obtained in A and emulsified to give a water-in-oil (W/O) cosmetic. The viscosities of Examples and Comparative Examples immediately after preparation were medium in the range of 20,000 to 40,000 mPa·s.

**[Table 2]**

| Composition (%) | | Example | | Comparative Example | |
|---|---|---|---|---|---|
| | | 4 | 5 | 6 | 7 |
| (1) | (a) Production Example 1 | 2 | - | 2 | - |
| | (a) Production Example 2 | - | 2 | - | 2 |
| | (b) KSG-710 (Note 1) | 3 | 3 | - | - |
| | KF-96A-6cs (Note 4) | 20 | 20 | 23 | 23 |
| (2) | Sodium chloride | 0.5 | 0.5 | 0.5 | 0.5 |
| | Ethanol | 5 | 5 | 5 | 5 |
| | Butylene Glycol | 5 | 5 | 5 | 5 |
| | Water | 64.5 | 64.5 | 64.5 | 64.5 |
| Total | | 100 | 100 | 100 | 100 |
| (a)/(b) | | 0.7 | 0.7 | - | - |
| Emulsion particle size | | A | B | A | B |
| Initial viscosity (mPa·s) | | 33,900 | 24,800 | 31,000 | 22,000 |
| Post-storage viscosity (mPa·s) | | 28,000 | 23,000 | 9,800 | 14,000 |
| Viscosity change (%) | | -17 | -7 | -68 | -36 |
| Stability | | B | A | D | D |

| | | | | | |
|---|---|---|---|---|---|
| Production Example 1: Aminoalcohol-modified silicone obtained in Production Example 1 Production Example 2: Aminoalcohol-modified silicone obtained in Production Example 2 (Note 1) Mixture of70-80% dimethicone (6 cs) + 20-30% (dimethicone/polyglycerin-3) crosspolymer, Shin-Etsu Chemical Co., Ltd. (Note 4) Dimethicone (6 cs), Shin-Etsu Chemical Co., Ltd. | | | | | |

As is clear from Table 2, the cosmetics of Examples 4 and 5 had a small emulsion particle size and had good stability in spite of the fact that the emulsions contained stability-deteriorating ethanol. Further Examples are described below. The following Examples also achieved similar emulsion particle sizes and viscosity stability to Examples 1 to 5.

### [Example 6] Water-in-oil sunscreen cream

| | Composition | % |
|---|---|---|
| 1. | Cyclopentasiloxane | 30 |
| 2. | Squalane | 2.5 |
| 3. | Aminoalcohol-modified silicone obtained in Production Example 1 | 1.5 |
| 4. | KF-6105 (Note 1) | 3 |
| 5. | Ethylhexyl methoxycinnamate | 7.5 |
| 6. | t-Butyl methoxydibenzoylmethane | 3 |
| 7. | Polysilicone-15 | 1 |
| 8. | Disteardimonium hectorite | 1 |
| 9. | Tocopherol acetate | 0.1 |
| 10. | Ethanol | 1 |
| 11. | Sodium citrate | 0.5 |
| 12. | Magnesium sulfate | 0.5 |
| 13. | Preservative | 0.3 |
| 14. | Water | 48.1 |
| | Total | 100.0 |

| | | |
|---|---|---|
| (Note 1) Lauryl polyglyceryl-3 polydimethylsiloxyethyl dimethicone, Shin-Etsu Chemical Co., Ltd. | | |

### (Production method)

A: Ingredients 1 to 9 were mixed uniformly.
B: Ingredients 10 to 13 were dissolved uniformly into ingredient 14.
C: While performing stirring, the mixture obtained in B was gradually added to the mixture obtained in A and emulsified to give a sunscreen cream.

The sunscreen cream obtained as described above had a very good feeling on use and was also excellent in stability without any changes due to temperature or aging.

### [Example 7] Water-in-oil lip cream

| | Composition | % |
|---|---|---|
| 1. | Dextrin (palmitate/ethylhexanoate) (Note 1) | 9 |
| 2. | Cyclopentasiloxane | 53 |
| 3. | KP-545 (Note 2) | 5 |
| 4. | KSG-43 (Note 3) | 8 |
| 5. | Aminoalcohol-modified silicone obtained in Production Example 1 | 0.5 |
| 6. | KF-6105 (Note 4) | 1.5 |
| 7. | Butylene Glycol | 5 |
| 8. | Water | 10.8 |
| 9. | Colorant | 1.2 |
| 10. | Titanium dioxide-coated mica | 6 |
| | Total | 100.0 |

| | | |
|---|---|---|
| (Note 1) Rheopearl TT, Chiba Flour Milling Co., Ltd. (Note 2) Solution of 70% cyclopentasiloxane + 30% (acrylates/dimethicone) copolymer, Shin-Etsu Chemical Co., Ltd. (Note 3) Mixture of 65-75% triethylhexanoin + 25-35% (vinyl dimethicone/lauryl dimethicone) crosspolymer, Shin-Etsu Chemical Co., Ltd. (Note 4) Lauryl polyglyceryl-3 polydimethylsiloxyethyl dimethicone, Shin-Etsu Chemical Co., Ltd. | | |

### (Production method)

A: Ingredient 9 was mixed with part of ingredient 2 and dispersed with a roller mill. The dispersion obtained was heated and mixed together with ingredient 1, the remainder of ingredient 2, and ingredients 3 to 6.
B: Ingredients 7 and 8 were heated and added to the mixture obtained in A. The resultant mixture was emulsified and then cooled.
C: Ingredient 10 was added to the emulsion obtained in B to give a water-in-oil lip cream.

The water-in-oil lip cream obtained spread lightly, was not sticky or oily, and formed a long-lasting film on the lips.

### [Example 8] Water-in-oil sunscreen milky lotion

| | Composition | % |
|---|---|---|
| 1. | KSG-710 (Note 1) | 3 |
| 2. | KSG-15 (Note 2) | 2 |
| 3. | Aminoalcohol-modified silicone obtained in Production Example 2 | 1 |
| 4. | Dimethicone 6 CS | 5 |
| 5. | Cyclopentasiloxane | 36 |
| 6. | Isotridecyl isononanoate | 4 |
| 7. | Hydrophobized microparticulate titanium dioxide | 15 |
| 8. | Hydrophobized microparticulate zinc oxide | 10 |
| 9. | Silica | 0.2 |
| 10. | DPG | 2 |
| 11. | Sodium citrate | 0.2 |
| 12. | Sodium chloride | 0.5 |
| 13. | Preservative | q.s. |
| 14. | Fragrance | q.s. |
| 15. | Water | Balance |
| | Total | 100.0 |

| | | |
|---|---|---|
| (Note 1) Mixture of 70-80% dimethicone + 20-30% (dimethicone/(PEG-10/15)) crosspolymer, Shin-Etsu Chemical Co., Ltd. (Note 2) Mixture of 90-96% cyclopentasiloxane + 4-10% (dimethicone/vinyl dimethicone) crosspolymer, Shin-Etsu Chemical Co., Ltd. (Note 3) PEG-9 polydimethylsiloxyethyl dimethicone, Shin-Etsu Chemical Co., Ltd. | | |

### (Production method)

A: Ingredients 7 and 8 were mixed with part of ingredient 5 and part of ingredient 15, and the mixture was uniformly dispersed with a bead mill.
B: Ingredients 1 to 4, the remainder of ingredient 5, and ingredients 6 and 9 were mixed uniformly.
C: Ingredients 10 to 13 and the remainder of ingredient 15 were mixed.
D: While performing stirring, the mixture obtained in C was added to the mixture obtained in B and emulsified. The mixture obtained in A and ingredient 14 were further added to give a water-in-oil sunscreen milky lotion.

The water-in-oil sunscreen milky lotion obtained as described above had a very good feeling on use and was also excellent in stability without any changes due to temperature or aging.

### [Example 9] Water-in-oil sunscreen cream

| | Composition | % |
|---|---|---|
| 1. | KSG-840 (Note 1) | 3 |
| 2. | KSG-43 (Note 2) | 3 |
| 3. | Aminoalcohol-modified silicone obtained in Production Example 3 | 1.5 |
| 4. | KF-56A (Note 3) | 11 |
| 5. | Ethylhexyl methoxycinnamate | 7 |
| 6. | KSP-100 (Note 4) | 2 |
| 7. | Xanthan gum | 0.3 |
| 8. | Dipropylene glycol | 5 |
| 9. | Glycerin | 3 |
| 10. | Methylparaben | 0.1 |
| 11. | Sodium citrate | 0.2 |
| 12. | Sodium chloride | 0.5 |
| 13. | Water | Balance |
| | Total | 100.0 |

| | | |
|---|---|---|
| (Note 1) Mixture of 65-75% squalane + 25-35% (lauryl dimethicone/polyglycerin-3) crosspolymer, Shin-Etsu Chemical Co., Ltd. (Note 2) Mixture of 65-75% triethylhexanoin + 25-35% (vinyl dimethicone/lauryl dimethicone) crosspolymer, Shin-Etsu Chemical Co., Ltd. (Note 3) Diphenylsiloxyphenyl trimethicone, Shin-Etsu Chemical Co., Ltd. (Note 4) (Vinyl dimethicone/methicone silsesquioxane) crosspolymer, Shin-Etsu Chemical Co., Ltd. | | |

### (Production method)

A: Ingredients 1 to 6 were mixed uniformly.
B: Ingredients 7 to 13 were mixed uniformly.
C: The mixture obtained in B was added to the mixture obtained in A and emulsified to give a water-in-oil sunscreen cream.

The water-in-oil sunscreen milky lotion obtained as described above had a very good feeling on use and was also excellent in stability without any changes due to temperature or aging.

### [Example 10] Water-in-oil foundation

| | Composition | % |
|---|---|---|
| 1. | KSG-710 (Note 1) | 3.5 |
| 2. | KSG-15 (Note 2) | 5 |
| 3. | Aminoalcohol-modified silicone obtained in Production Example 4 | 2 |
| 4. | Disteardimonium hectorite | 1.2 |
| 5. | Triethylhexanoin | 5 |
| 6. | Dimethicone 6 CS | 6.5 |
| 7. | Cyclopentasiloxane | 22.6 |
| 8. | KP-578 (Note 3) | 0.5 |
| 9. | KTP-09W (Note 4) | q.s. |
| 10. | KTP-09R (Note 4) | q.s. |
| 11. | KTP-09Y (Note 4) | q.s. |
| 12. | KTP-09B (Note 4) | q.s. |
| 13. | Butylene Glycol | 5 |
| 14. | Sodium citrate | 0.2 |
| 15. | Sodium chloride | 0.5 |
| 16. | Water | 38 |
| | Total | 100.0 |

| | | |
|---|---|---|
| (Note 1) Mixture of 70-80% dimethicone + 20-30% (dimethicone/(PEG-10/15)) crosspolymer, Shin-Etsu Chemical Co., Ltd. (Note 2) Mixture of 90-96% cyclopentasiloxane + 4-10% (dimethicone/vinyl dimethicone) crosspolymer, Shin-Etsu Chemical Co., Ltd. (Note 3) (Acrylates/ethylhexyl acrylate/dimethicone methacrylate) copolymer, Shin-Etsu Chemical Co., Ltd. (Note 4) KF-9909-treated coloring inorganic pigments, W: white, R: red, Y: yellow, B: black, Shin-Etsu Chemical Co., Ltd. | | |

### (Production method)

A: Ingredients 8 to 12 and part of ingredient 7 were uniformly dispersed with a roll mill.
B: Ingredients 1 to 6 and the remainder of ingredient 7 were mixed uniformly.
C: Ingredients 13 to 16 were mixed uniformly.
D: The mixture obtained in C was added to the mixture obtained in B and emulsified. The mixture obtained in A was further added and mixed to give a water-in-oil foundation.

The water-in-oil foundation obtained as described above had a very good feeling on use and was also excellent in stability without any changes due to temperature or aging.

### [Example 11] Water-in-oil stick foundation

| | Composition | % |
|---|---|---|
| 1. | KSG-710 | 4 |
| 2. | Aminoalcohol-modified silicone obtained in Production Example 5 | 1.5 |
| 3. | Inulin stearate (Note 3) | 2 |
| 4. | Ceresin | 6 |
| 5. | KF-56A (Note 4) | 11.5 |
| 6. | Ethylhexyl methoxycinnamate | 5 |
| 7. | KMP-590 (Note 5) | 1.5 |
| 8. | Isotridecyl isononanoate | 4 |
| 9. | KF-6105 (Note 6) | 1 |
| 10. | KTP-09W (Note 7) | 6.5 |
| 11. | KTP-09R (Note 7) | q.s. |
| 12. | KTP-09Y (Note 7) | q.s. |
| 13. | KTP-09B (Note 7) | q.s. |
| 14. | DPG | 6 |
| 15. | Methylparaben | 0.1 |
| 16. | Sodium citrate | 0.2 |
| 17. | Sodium chloride | 0.5 |
| 18. | Water | Balance |
| | Total | 100.0 |

| | | |
|---|---|---|
| (Note 1) Mixture of 70-80% dimethicone + 20-30% (dimethicone/polyglycerin-3) crosspolymer, Shin-Etsu Chemical Co., Ltd. (Note 2) Lauryl PEG-9 polydimethylsiloxyethyl dimethicone, Shin-Etsu Chemical Co., Ltd. (Note 3) Rheopearl ISK2, Chiba Flour Milling Co., Ltd. (Note 4) Diphenylsiloxyphenyl trimethicone, Shin-Etsu Chemical Co., Ltd. (Note 5) Polymethylsilsesquioxane, Shin-Etsu Chemical Co., Ltd. (Note 6) Lauryl polyglyceryl-3 polydimethylsiloxyethyl dimethicone, Shin-Etsu Chemical Co., Ltd. (Note 7) KF-9909-treated coloring inorganic pigments, W: white, R: red, Y: yellow, B: black, Shin-Etsu Chemical Co., Ltd. | | |

### (Production method)

A: Ingredients 8 to 13 were dispersed with a roll mill.
B: The dispersion obtained in A and ingredients 1 to 7 were heated to 95°C and mixed uniformly.
C: Ingredients 14 to 18 were mixed uniformly and heated to 85°C.
D: The mixture obtained in C was added to the mixture obtained in B and emulsified. The emulsion obtained was poured into a stick container and slowly cooled to give a stick foundation.

The water-in-oil stick foundation obtained as described above had a good feeling on use, spread well, and exhibited good cosmetic durability and formulation stability.

### [Example 12] Polyhydric alcohol-in-oil emulsion cream

| | Composition | % |
|---|---|---|
| 1. | KSG-710 (Note 1) | 5 |
| 2. | KSG-15 (Note 2) | 20 |
| 3. | KSG-16 (Note 3) | 35 |
| 4. | Aminoalcohol-modified silicone obtained in Production Example 6 | 1 |
| 5. | Cyclopentasiloxane | 5 |
| 6. | KF-7312J (Note 4) | 4 |
| 7. | KSP-300 (Note 5) | 5 |
| 8. | Preservative | q.s. |
| 9. | Fragrance | q.s. |
| 10. | Glycerin | 5 |
| 11. | Butylene Glycol | 10 |
| | Total | 100.0 |

| | | |
|---|---|---|
| (Note 1) Mixture of 70-80% dimethicone + 20-30% (dimethicone/polyglycerin-3) crosspolymer, Shin-Etsu Chemical Co., Ltd. (Note 2) Mixture of 90-96% cyclopentasiloxane + 4-10% (dimethicone/vinyl dimethicone) crosspolymer, Shin-Etsu Chemical Co., Ltd. (Note 3) Mixture of 70-80% dimethicone + 20-30% (dimethicone/vinyl dimethicone) crosspolymer, Shin-Etsu Chemical Co., Ltd. (Note 4) Cyclopentasiloxane solution of 50% trimethylsiloxysilicic acid, Shin-Etsu Chemical Co., Ltd. (Note 5) (Diphenyl dimethicone/vinyl diphenyl dimethicone/silsesquioxane) crosspolymer, Shin-Etsu Chemical Co., Ltd. | | |

### (Production method)

A: Ingredients 1 to 9 were admixed uniformly.
B: Ingredients 8, 10, and 11 were mixed.
C: The mixture obtained in B was added to the mixture obtained in A and emulsified uniformly to give a cream.

The polyhydric alcohol-in-oil emulsion cream obtained as described above spread lightly, was not sticky or oily, and had a moist feel.

### [Example 13] Water-in-oil cream

| | Composition | % |
|---|---|---|
| 1. | KSG-710 (Note 1) | 4 |
| 2. | Aminoalcohol-modified silicone obtained in Production Example | 7 0.5 |
| 3. | Cyclopentasiloxane | 8.8 |
| 4. | KSP-101 (Note 2) | 2 |
| 5. | Butylene Glycol | 5 |
| 6. | Diglycerin | 5 |
| 7. | PEG-12 | 15 |
| 8. | Phenoxyethanol | 0.3 |
| 9. | Chili pepper extract | q.s. |
| 10. | Glycerin | Balance |
| | Total | 100.0 |

| | | |
|---|---|---|
| (Note 1) Mixture of 70-80% dimethicone + 20-30% (dimethicone/polyglycerin-3) crosspolymer, Shin-Etsu Chemical Co., Ltd. (Note 2) (Vinyl dimethicone/methicone silsesquioxane) crosspolymer, Shin-Etsu Chemical Co., Ltd. | | |

### (Production method)

A: Ingredients 1 to 4 were mixed uniformly.
B: Ingredients 5 to 10 were mixed uniformly.
C: The mixture obtained in B was added to the mixture obtained in A and emulsified to give a water-in-oil cream.

The water-in-oil cream obtained as described above had a very good feeling on use and was also excellent in stability without any changes due to temperature or aging.

### [Example 14] Oil-in-water cream

| | Composition | % |
|---|---|---|
| 1. | KSG-15 (Note 1) | 8.0 |
| 2. | KSG-16 (Note 2) | 30.0 |
| 3. | Cyclopentasiloxane | 10.0 |
| 4. | Butylene Glycol | 3.0 |
| 5. | KF-6100 (Note 3) | 0.6 |
| 6. | Aminoalcohol-modified silicone obtained in Production Example 8 | 0.3 |
| 7. | Aqueous (acryloyldimethyltaurate ammonium/VP) copolymer solution (Note 4) | 13.0 |
| 8. | Aqueous (acrylamide/sodium acryloyldimethyltaurate) copolymer/ isohexadecane/Polysorbate 80 solution (Note 5) | 0.6 |
| 9. | 1% Aqueous sodium chloride solution | 8.0 |
| 10. | Water | 26.5 |
| | Total | 100.0 |

| | | |
|---|---|---|
| (Note 1) Mixture of 90-96% cyclopentasiloxane + 4-10% (dimethicone/vinyl dimethicone) crosspolymer, Shin-Etsu Chemical Co., Ltd. (Note 2) Mixture of 70-80% dimethicone + 20-30% (dimethicone/vinyl dimethicone) crosspolymer, Shin-Etsu Chemical Co., Ltd. (Note 3) Polyglyceryl-3 disiloxane dimethicone, Shin-Etsu Chemical Co., Ltd. (Note 4) Aristoflex AVC, Clariant (Note 5) Simulgel 600, Seppic | | |

### (Production method)

A: Ingredients 4 to 10 were mixed uniformly.
B: Ingredients 1 to 3 were mixed uniformly.
C: The mixture obtained in B was added to the mixture obtained in A and emulsified to give an oil-in-water cream.

The water-in-oil cream obtained as described above had a very good feeling on use and was also excellent in stability without any changes due to temperature or aging.

### [Example 15] Lipstick

| | Composition | % |
|---|---|---|
| 1. | Candelilla wax | 4 |
| 2. | Polyethylene | 2 |
| 3. | Microcrystalline wax | 3 |
| 4. | Ceresin | 7 |
| 5. | KP-561P (Note 1) | 15 |
| 6. | KF-6105 (Note 2) | 2 |
| 7. | Aminoalcohol-modified silicone obtained in Production Example 2 | 1 |
| 8. | Macadamia seed oil | 28 |
| 9. | Diisostearyl malate | 1 |
| 10. | Hydrogenated polyisobutene | 19 |
| 11. | Isotridecyl isononanoate | 18 |
| 12. | Pigment | q.s. |
| 13. | Mica | q.s. |
| | Total | 100.0 |

| | | |
|---|---|---|
| (Note 1) (Acrylates/stearyl acrylate/dimethicone methacrylate) copolymer, Shin-Etsu Chemical Co., Ltd. (Note 2) Lauryl polyglyceryl-3 polydimethylsiloxyethyl dimethicone, Shin-Etsu Chemical Co., Ltd. | | |

### (Production method)

A: Ingredients 1 to 11 were heated to 90°C and mixed uniformly.
B: Ingredients 12 and 13 were mixed uniformly with the mixture obtained in A at 80°C. The resultant mixture was poured into a stick container and slowly cooled to give a lipstick.

The lipstick obtained as described above spread lightly, was not sticky or oily, and formed a long-lasting film on the lips.

### [Example 16] Sunscreen milky lotion (shaking)

| | Composition | % |
|---|---|---|
| 1. | Cyclopentasiloxane | 23.0 |
| 2. | KF-56A (Note 1) | 5.5 |
| 3. | Triethylhexanoin | 5 |
| 4. | KSG-18A (Note 2) | 3 |
| 5. | Aminoalcohol-modified silicone obtained in Production Example 1 | 1 |
| 6. | KF-6105 (Note 3) | 1 |
| 7. | Ethylhexyl methoxycinnamate | 7.5 |
| 8. | Diethylamino hydroxybenzoyl hexyl benzoate | 1 |
| 9. | Octocrylene | 2.5 |
| 10. | KSP-105 (Note 4) | 0.5 |
| 11. | SPD-T5 (Note 5) | 5 |
| 12. | SPD-Z5 (Note 6) | 10 |
| 13. | Butylene Glycol | 3 |
| 14. | Sodium citrate | 0.2 |
| 15. | Sodium chloride | 0.5 |
| 16. | Ethanol | 6 |
| 17. | Water | 25.3 |
| | Total | 100.0 |

| | | |
|---|---|---|
| (Note 1) Diphenylsiloxyphenyl trimethicone, Shin-Etsu Chemical Co., Ltd. (Note 2) Mixture of 80-90% diphenylsiloxyphenyl trimethicone + 10-20% (dimethicone/phenyl vinyl dimethicone) crosspolymer, Shin-Etsu Chemical Co., Ltd. (Note 3) Lauryl polyglyceryl-3 polydimethylsiloxyethyl dimethicone, Shin-Etsu Chemical Co., Ltd. (Note 4) (Vinyl dimethicone/methicone silsesquioxane) crosspolymer, Shin-Etsu Chemical Co., Ltd. (Note 5) Cyclopentasiloxane dispersion of 40% microparticulate titanium dioxide, Shin-Etsu Chemical Co., Ltd. (Note 6) Cyclopentasiloxane dispersion of 60% microparticulate zinc oxide, Shin-Etsu Chemical Co., Ltd. | | |

### (Production method)

A: Ingredients 1 to 9 were mixed uniformly.
B: Ingredient 10 was added to the mixture obtained in A and dispersed uniformly.
C: Ingredients 13 to 16 were added to ingredient 17 to give a solution.
D: The mixture obtained in C was gradually added to the mixture obtained in B and emulsified. Ingredients 11 and 12 were further added to give a sunscreen milky lotion.

The sunscreen milky lotion obtained as described above spread lightly, gave a dry and non-sticky finish, and was free of changes due to temperature or aging, thus being shown to be superior in usability and stability.

### [Example 17] Water-breaking W/O foundation

| | Composition | % |
|---|---|---|
| 1. | KSG-710 (Note 1) | 4 |
| 2. | KSG-18A (Note 2) | 1 |
| 3. | Aminoalcohol-modified silicone obtained in Production Example 2 | 0.1 |
| 4. | Ethylhexyl methoxycinnamate | 7.5 |
| 5. | Dimethylpolysiloxane (2 cs) | 5.6 |
| 6. | Cyclopentasiloxane | 4.8 |
| 7. | KF-6115 (Note 3) | 0.3 |
| 8. | KTP-09W (Note 4) | 5.1 |
| 9. | KTP-09Y (Note 4) | 0.58 |
| 10. | KTP-09R (Note 4) | 0.25 |
| 11. | KTP-09B (Note 4) | 0.07 |
| 12. | Hydrophobized microparticulate zinc oxide (Note 5) | 6 |
| 13. | Butylene Glycol | 8 |
| 14. | Sodium citrate | 0.2 |
| 15. | Magnesium sulfate | 0.5 |
| 16. | Water | 56 |
| | Total | 100.0 |

| | | |
|---|---|---|
| (Note 1) Mixture of 70-80% dimethicone (6 cs) + 20-30% (dimethicone/polyglycerin-3) crosspolymer, Shin-Etsu Chemical Co., Ltd. (Note 2) Mixture of 80-90% diphenylsiloxyphenyl trimethicone + 10-20% (dimethicone/phenyl vinyl dimethicone) crosspolymer, Shin-Etsu Chemical Co., Ltd. (Note 3) Lauryl polyglyceryl-3 polydimethylsiloxyethyl dimethicone, Shin-Etsu Chemical Co., Ltd. (Note 4) KF-9909-treated coloring inorganic pigments, W: white, R: red, Y: yellow, B: black, Shin-Etsu Chemical Co., Ltd. (Note 5) Treated with AES-3083, Shin-Etsu Chemical Co., Ltd. | | |

### (Production method)

| | |
|---|---|
| Preparation of oil phase: | Ingredients 1 to 5 were mixed uniformly. |
| Preparation of aqueous phase: | Ingredients 13 to 16 were mixed uniformly. |
| Preparation of paste phase: | Ingredients 8 to 12 were mixed together in ingredients 6 and 7 and dispersed with a three-roll mill. |

The aqueous phase was gently added to the oil phase to effect a phase change. After emulsification, the paste was mixed. A water-breaking W/O foundation was thus obtained.

The water-breaking W/O foundation obtained was shown to have a good feeling on use, a good water-breaking feel, and good applicability.

### [Example 18] W/O cosmetic stick

| | Composition | % |
|---|---|---|
| 1. | KSG-830 (Note 1) | 5 |
| 2. | KSG-43 (Note 2) | 5 |
| 3. | Aminoalcohol-modified silicone obtained in Production Example 9 | 0.5 |
| 4. | Meadowfoam oil | 2 |
| 5. | KF-56A (Note 3) | 8 |
| 6. | Argania spinosa kernel oil | 0.5 |
| 7. | Ceresin | 10 |
| 8. | Butylene Glycol | 7 |
| 9. | PCA-Na | 2 |
| 10. | Diglycerin | 3 |
| 11. | Sodium citrate | 0.2 |
| 12. | Magnesium sulfate | 1 |
| 13. | Water | Ba lance |
| | Total | 100.0 |

| | | |
|---|---|---|
| (Note 1) Mixture of 75-85% triethylhexanoin + 15-25% (lauryl dimethicone/polyglycerin-3) crosspolymer, Shin-Etsu Chemical Co., Ltd. (Note 2) Mixture of 65-75% triethylhexanoin + 25-35% (vinyl dimethicone/lauryl dimethicone) crosspolymer, Shin-Etsu Chemical Co., Ltd. (Note 3) Diphenylsiloxyphenyl trimethicone, Shin-Etsu Chemical Co., Ltd. | | |

### (Production method)

A: Ingredients 1 to 7 were mixed uniformly at 90°C.
B: Ingredients 8 to 13 were mixed uniformly at 85°C.
C: The mixture obtained in B was added to the mixture obtained in A and emulsified. The emulsion was poured into a stick container to give a W/O cosmetic stick.

The W/O cosmetic stick obtained had a good feeling on use, good applicability, and excellent storage stability.

### [Example 19] Hair oil

| | Composition | % |
|---|---|---|
| 1. | Aminoalcohol-modified silicone obtained in Production Example 11 | 3 |
| 2. | KF-56A (Note 1) | 7 |
| 3. | Diethylhexyl succinate | 10 |
| 4. | X-21-5613 (Note 2) | 1.5 |
| 5. | Tocopherol | 0.1 |
| 6. | KSG-820Z (Note 3) | 1.5 |
| 7. | Fragrance | 0.1 |
| 8. | Light liquid isoparaffin | Balance |
| | Total | 100.0 |

| | | |
|---|---|---|
| (Note 1) Diphenylsiloxyphenyl dimethicone (labeling name), Shin-Etsu Chemical Co., Ltd. (Note 2) Dimethiconol (labeling name), Shin-Etsu Chemical Co., Ltd. (Note 3) Polyglycerin-modified silicone mixture in which silicone chains and alkyl chains are branched from a crosslinked silicone backbone, Shin-Etsu Chemical Co., Ltd. | | |

### (Production method)

Ingredients 1 to 8 were mixed uniformly to give a hair oil.

The hair oil obtained had a good feeling on use, good applicability, and excellent storage stability.

### [Example 20] Hair treatment

| | Composition | % |
|---|---|---|
| 1. | Aminoalcohol-modified silicone obtained in Production Example 10 | 1 |
| 2. | Cetanol | 2 |
| 3. | Cetyl octanoate | 3 |
| 4. | Butyl p-hydroxybenzoate | 0.1 |
| 5. | KF-56A (Note 1) | 1 |
| 6. | Behentrimonium chloride | 1 |
| 7. | Propylene glycol | 5 |
| 8. | Hydroxyethyl cellulose | 0.1 |
| 9. | Water | Balance |
| 10. | X-52-2328 (Note 2) | 4 |
| 11. | KF-6100 (Note 3) | 0.5 |
| 12. | Fragrance | q.s. |
| | Total | 100.0 |

| | | |
|---|---|---|
| (Note 1) Diphenylsiloxyphenyl dimethicone (labeling name), Shin-Etsu Chemical Co., Ltd. (Note 2) Aminopropyl dimethicone (labeling name), Shin-Etsu Chemical Co., Ltd. (Note 3) Polyglyceryl-3 disiloxane dimethicone, Shin-Etsu Chemical Co., Ltd. | | |

### (Production method)

A: Ingredients 6 to 9 were heated to 70°C and mixed uniformly.
B: Ingredients 1 to 5 were heated to 70°C and mixed uniformly.
C: The mixture obtained in B was added to the mixture obtained in A and emulsified. After the emulsion was slowly cooled, ingredients 10 to 12 were added. A hair treatment was thus obtained.

The hair treatment obtained had a good feeling on use, good applicability, and excellent storage stability.

## Claims

1. A cosmetic comprising:
(a) an aminoalcohol-modified silicone and
(b) a polyglycerin-modified silicone surfactant.

2. The cosmetic according to claim 1, wherein the aminoalcohol-modified silicone (a) is an aminoalcohol-modified silicone represented by formula (1) below: 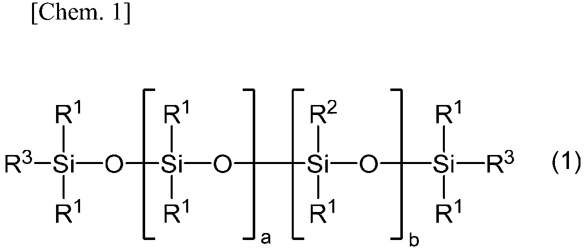
wherein R¹ independently at each occurrence is a monovalent hydrocarbon group selected from C1-C20 alkyl groups, C6-C15 aryl groups, and C7-C15 aralkyl groups; R² independently at each occurrence is a group represented by formula (2) below: 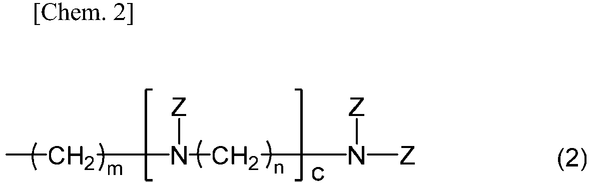
wherein Z independently at each occurrence is a group selected from hydrogen atom, -CH₂CH(OH)CH₃, and -CH(CH₃)CH₂OH, 50 mol% or more of a number of moles of all Zs in the molecule are groups selected from -CH₂CH(OH)CH₃ and -CH(CH₃)CH₂OH, m is an integer of 1 to 10, and n is an integer of 1 to 5; R³ is R¹ or R²; a is an integer of 1 to 100; b is an integer of 0 to 10; c is an integer of 0 to 4; and when b = 0, at least one of R³s is R².

3. The cosmetic according to claim 2, wherein in formula (1), R² is selected from aminoalcohol groups represented by formulas (3) and (4) below: 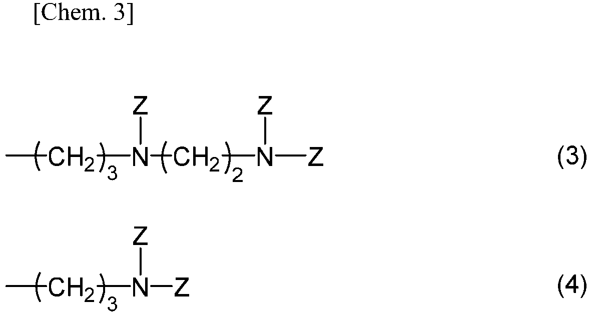 wherein Z is the same as defined above.

4. The cosmetic according to claim 2, wherein in formula (1), b is an integer of 1 to 10, and a/b is 10 to 30.

5. The cosmetic according to claim 2, wherein in formula (1), 99 to 100 mol% of the number of moles of all Zs in the molecule are -CH₂CH(OH)CH₃ or -CH(CH₃)CH₂OH.

6. The cosmetic according to any one of claims 1 to 5, wherein the ingredient (b) is a partially crosslinked polyglycerin-modified silicone surfactant.
